# EUROPEAN PATENT SPECIFICATION

(11) **EP 3 087 392 B1**
(45) Date of publication and mention of the grant of the patent: **31.10.2018**
(21) Application number: 14874414.7
(22) Date of filing: 23.12.2014
(51) Int. Cl.: G01N 33/68

(54) **MENTAL ILLNESS MODEL AND MENTAL ILLNESS RISK ASSESSMENT TEST FOR SCHIZOPHRENIC PSYCHOSIS**
GEISTESKRANKHEITSMODELL UND GEISTESKRANKHEITSRISIKOBEURTEILUNGSTEST FÜR PSYCHOSE AUS DEM SCHIZOPHRENEN FORMENKREIS
MODÈLE DE MALADIE MENTALE ET TEST D'ÉVALUATION DU RISQUE DE DÉVELOPPER UNE MALADIE MENTALE POUR LA PSYCHOSE SCHIZOPHRÉNIQUE

(30) Priority: 23.12.2013 AU 2013905047; 04.06.2014 AU 2014902139; 23.09.2014 AU 2014903799
(43) Date of publication of application: 02.11.2016
(73) Proprietor: Williams, Stephanie Sue, Norwood, South Australia 5067 (AU)
(72) Inventor: WILLIAMS, Stephanie Sue, Norwood, South Australia 5067 (AU); RADCLIFFE, Natasha Jane, Grange, South Australia 5022 (AU)
(74) Representative: Kilger, Ute
(86) International application number: PCT/AU2014/050444
(87) International publication number: WO 2015/095930

(56) References cited:
- WO-A1-2010/041068
- WO-A1-2010/041068
- WO-A1-2010/066000
- WO-A1-2010/066000
- WO-A1-2013/186562
- WO-A1-2013/186562
- WO-A2-03/042654
- CYNTHIA S. WEICKERT ET AL: "Biomarkers in Schizophrenia: A Brief Conceptual Consideration", DISEASE MARKERS., vol. 35, no. 1, 1 January 2013 (2013-01-01), pages 3-9, XP055354941, GB ISSN: 0278-0240, DOI: 10.1155/2013/510402
- STEPHANIE FRYAR-WILLIAMS ET AL: "Biomarker Symptom Profiles for Schizophrenia and Schizoaffective Psychosis", OPEN JOURNAL OF PSYCHIATRY, vol. 05, no. 01, 28 January 2015 (2015-01-28), pages 78-112, XP055354948, ISSN: 2161-7325, DOI: 10.4236/ojpsych.2015.51011
- STEPHANIE FRYAR-WILLIAMS ET AL: "Biomarkers of a five-domain translational substrate for schizophrenia and schizoaffective psychosis", BIOMARKER RESEARCH, vol. 49, no. 12, 1 January 2015 (2015-01-01), page 975, XP055180378, DOI: 10.1186/s40364-015-0028-1
- FOUNDATION NEWS ET AL.: 'Volunteers Needed for Mental Health Study' 15 November 2011, XP008185908 Retrieved from the Internet: <URL:http://www.hospitalresearch.com.au/vol unteers-needed-for-mental-health-study> [retrieved on 2015-01-29]
- WEICKERT, C. S. ET AL.: 'Biomarkers in schizophrenia: a brief conceptual consideration' DISEASE MARKERS vol. 35, no. 1, 01 January 2013, pages 3 - 9, XP055354941 DOI: 10.1155/2013/510402
- FRYAR-WILLIAMS, S. ET AL.: 'Biomarker symptom profiles for schizophrenia and schizoaffective psychosis' OPEN JOURNAL OF PSYCHIATRY vol. 5, no. 1, 2015, pages 78 - 112, XP055354948 DOI: 10.4236/OJPSYCH.2015.51011 Retrieved from the Internet: <URL:http://dx.doi.org/10.4236/ojpsych.2015 .51011> [retrieved on 2015-01-29]

## Description

### Field of the Invention

The present invention relates to a novel set of biomarkers for the diagnosis of, and prediction of susceptibility to, schizophrenia, schizo-affective disorder and psychosis and to methods for the diagnosis of, and prediction of susceptibility to, schizophrenia, schizo-affective disorder and psychosis employing these biomarkers.

### Background of the Invention

The incidence of mental illness, and its impact on society, appears to be increasing. Because of the immense personal, social and financial impact of mental illness on sufferers, their families, the community, the health system and the economy, the ability to accurately diagnose mental illness is of critical importance. Schizophrenia is one of the most disabling mental illnesses, with a lifetime prevalence of about one-percent in the population. Because schizophrenia usually appears early in life and is often chronic, the costs of the disorder are substantial.

Symptoms associated with schizophrenia are typically characterized as falling into two broad categories--positive and negative (or deficit) symptoms--with a third category, disorganized, recently added. Positive symptoms include delusions and hallucinations. Negative symptoms include restricted range and intensity of emotional expression and reduced thought and speech productivity. More recently, a third category of symptom, disorganised, has been recognized. Disorganised symptoms include disorganised speech, disorganised behavior and poor attention. According to Diagnostic and Statistical Manual of Mental Disorder-IV (DSM-IV) (and the current DSM V), the essential features of schizophrenia consist of a mixture of characteristic signs and symptoms that have been present for a significant length of time during a 1-month period with some signs of the disorder persisting for at least 6 months. However no single symptom is characteristic of the disease. Moreover, recognition of the heterogeneity of schizophrenia and psychosis has led to increasing dissatisfaction with currently used classification systems.

Current diagnostic approaches are typically descriptive or rely predominantly on symptomatic analyses based on, for example, physical examination, gross medical evaluation, psychological and/or psychiatric evaluation, anecdotal family history, and emotional history. There is a clear need for improved, objective, neuroscience-based methods for diagnosing schizophrenia and psychosis. This would be greatly facilitated by the identification of markers schizophrenia and psychosis enabling the development of accurate, sensitive and easy to employ diagnostic tests.

Weickert et al. ("Biomarkers in schizophrenia: a brief conceptual consideration", Disease markers, vol. 35, no. 1, (2013-01-01), p. 3-9) discloses a review of biomarker in Schizophrenia, according to which no biomarker currently exists for Schizophrenia.

WO 20013/186562 discloses a method of diagnosing psychotic disorders, including Schizophrenia comprising quantifying the amounts of different biomarkers such as interleukin 10 (IL-10), interleukin 18 (IL-18), interleukin 8 (IL-8), monocyte chemotactic protein 1 (MCP-1), macrophage inflammatory protein 1 beta (MIP-113), matrix metalloproteinase 2 (MMP), tumor necrosis factor beta (TNF-13), interleukin 4 (IL-4) and interferon gamma (IFN-γ.

WO 2010/041068 describes a method for diagnosis of Schizophrenia or psychotic disorders by detecting biomarkers such as dopamine hydroxylase, proinsulin, insulin, C-peptide, chromogranin A or VGF.

WO2010/066000 describes the diagnosis of a mental or neurodegenerative disorder such as Schizophrenia or psychosis comprising determining the levels of one or more biomarkers such as pyrroles, histamine, methionine adenosyltransferase (MAT) activity, homocysteine, copper and zinc.

Advances in medication have raised outcome expectations for schizophrenia, however a reduction in symptoms is far less-often accompanied by restoration of function and quality of life, despite the many psychosocial interventions employed. There are clear implications that other factors must contribute to this sustained reduction of functional restoration. The novel mental illness model described herein identifies some of these unmet-need factors. It also provides a link between specifically problematic symptoms and behaviours of schizophrenia and their specific biological underpinnings, so that management of difficult behaviour and distressing mental illness symptoms can be more clearly understood and more comprehensively managed.

### Summary of the Invention

According to a first aspect of the present invention there is provided a method for diagnosing schizophrenia, schizo-affective disorder and/or psychosis in an individual or predicting risk of the individual developing schizophrenia, schizo-affective disorder or psychosis, the method comprising
(i) determining values for one or more markers in each of five domains in one or more biological samples obtained from the individual:
   (a) a neurotransmitter domain (also referred to herein as the catecholamine domain) comprising dopamine, noradrenaline and adrenaline;
   (b) an oxidative stress domian comprising urinary hydroxyhemopyrroline-2-one (HPL) and urinary creatinine or other marker of oxidative stress;
   (c) a nutrition-biochemistry domain comprising free copper to zinc ratio, activated vitamin B6, red cell folate, serum vitamin B12, and vitamin D;
   (d) a visual processing domain comprising visual span, visual speed of processing discrepancy, visual speed of processing, and distance vision predominantly on the right; and
   (e) an auditory processing domain comprising reverse digit span, competing words discrepancy, auditory speed of processing discrepancy, and auditory speed of processing;
   and
(ii) comparing values for said one or more markers in each of said domains to control values of said markers in subjects not suffering from schizophrenia, schizo-affective disorder or psychosis, wherein the values of said markers indicative of schizophrenia or psychosis are, relative to said control values:
   - in the neurotransmitter domain, high dopamine, high noradrenaline, and high adrenaline;
   - in the oxidative stress domain, high urinary hydroxyhemopyrroline-2-one divided by urinary creatinine;
   - in the nutrition-biochemistry domain, high free copper to zinc ratio (or low zinc to free copper ratio), low activated vitamin B6, low red cell folate, high serum vitamin B12, and low vitamin D;
   - in the visual processing domain, low visual span, high visual speed of processing discrepancy (as percentage of age), low visual speed of processing (percentile), and poor distance vision, particularly on right; and
   - in the auditory processing domain, low reverse digit span, high competing words discrepancy (as percentage of pass score), high auditory speed of processing discrepancy (as percentage of age), and low auditory speed of processing (percentile).

The psychosis may be schizophrenic psychosis or schizo-affective psychosis.

Typically the method comprises determining values for each of the markers in each of the domains as defined above.

Optionally, the method further comprises determining values for one or more markers in a middle ear domain comprising threshold ear canal volume, threshold peak middle ear pressure, threshold gradient middle ear pressure, threshold stapes amplitude projected, threshold time to offset over baselength and threshold percentage baselength over duration, and comparing values for said one or more markers to control values of said markers in subjects not suffering from schizophrenia, schizo-affective disorder or psychosis, wherein the values of said markers indicative of schizophrenia, schizo-affective disorder or psychosis are, relative to said control values, high threshold ear canal volume, low threshold peak middle ear pressure, high threshold gradient middle ear pressure, high threshold stapes amplitude projected, low threshold time to offset over baselength and high threshold percentage baselength over duration.

In particular embodiments, said method comprises conducting statistical analysis of determined values of said markers in combination and diagnosing schizophrenia, schizo-affective disorder or psychosis in said individual on the basis of the combined analysis. Typically said statistical analysis comprises receiver operating characteristic (ROC) analysis, and optionally odds-ratio calculation or regression analysis. Said ROC analysis may comprise ascertaining ROC ranges for individual ROC variables and summated sets of ROC variables and summated sets of ROC domain scores, based on ROC cut-off values, using an appropriate means to determine proxy standard deviation for ROC cut-off values adjusted for their position in the distribution of the variable. Odds ratio and regression analysis may be performed on summated ROC scores of multiple ROC domains to diagnose or determine risk prediction.

Biological samples obtained from the individual to determine marker levels for the neurotransmitter domain, the oxidative stress domain and the nutrition-biochemistry domain may be derived from any suitable body fluid or tissue. For example the sample may comprise blood (such as erythrocytes, leukocytes, whole blood, blood plasma or blood serum), saliva, sputum, urine, breath, condensed breath, amniotic fluid, cerebrospinal fluid or tissue (post-mortem or living, fresh or frozen). In a particular embodiment the sample comprises whole blood, blood serum or urine.

The markers in the neurotransmitter domain and the nutrition-biochemistry domain are typically determined from blood or urine samples obtained from the individual, more typically from blood samples. The markers in the oxidative stress domain are typically determined from urine samples obtained from the individual.

The method may further comprise the determination of levels of one or more additional markers in a sample derived from said individual. By way of example only, additional markers measured may include abnormal threshold visual speed of processing performance (identified by increased interstimulus interval (ISI) required for correct visual order processing in msecs), abnormal competing words (dichotic listening) performance (identified as low score on SCAN C competing words test or other dichotic listening test), abnormal threshold auditory speed of processing (identified by increased (ISI) required for correct auditory order processing in msecs), urinary hydroxyhemopyrroline-2-one (HPL) adjusted for urine creatinine or specific gravity of urine, methyl malonic acid, vitamin B2 (riboflavin), riboflavin cofactor 420, L-threonine, 5-methyltetrahydrofolate, S-adenosylmethionine (SAMe), S-adenosylhomocysteine (SAH), reduced glutathione and oxidised glutathione, wherein abnormal levels of methyl malonic acid, low vitamin B2, low 5-methyltetrahydrofolate, low or high SAMe, high SAH (and low or high SAMe:SAH ratio), low reduced glutathione and high oxidised glutathione in a sample obtained from the individual, compared to levels in subjects not suffering from schizophrenia, schizo-affective disorder or psychosis, are suggestive of schizophrenia, schizo-affective disorder or psychosis.

Another aspect of the invention provides a method for evaluating the efficacy of a treatment regime in a subject suffering from schizophrenia, schizo-affective disorder and/or psychosis, the method comprising:
(a) treating the subject with a treatment regime for a period sufficient to evaluate the efficacy of the regime;
(b) obtaining one or more biological samples from the subject;
(c) determining the levels of a panel of markers in the sample(s) in accordance with the above-described first aspect;
(d) repeating steps (b) and (c) at least once over a period of time; and
(e) determining whether the marker levels changes over the period of time.

Disease control in the subject may then be improved by adjusting the timing, frequency and/or intensity of marker testing and /or adjusting the identity, timing and/or intensity of a treatment regime to thereby normalise the levels of one or more of the markers.

Another aspect of the invention provides a method for designing a suitable treatment regime for a subject suffering from schizophrenia, schizo-affective disorder and/or psychosis, the method comprising monitoring the levels of a panel of markers in the subject in accordance with the above described first aspect, in the presence or absence of a treatment regime for treating the schizophrenia, schizo-affective disorder and/or psychosis and adjusting the identity, timing and/or intensity of the treatment regime so as to normalise the levels of one or more of the markers.

Also provided is a method for treating a subject suffering from schizophrenia, schizo-affective disorder and/or psychosis, comprising administering to the subject a treatment regime designed according to the above aspect.

Methods embodied by the above described aspects and embodiments of the invention are particularly suitable for diagnosing and evaluating the status of schizophrenia, schizo-affective disorder and/or psychosis in human subjects. However, the invention is not limited thereto and extends to any mammal, for example mammals useful as a model for said disorders in humans. Typically the subject is a mammal, more typically a human. The subject may be of any age, child, adolescent, adult or elderly.

### Brief Description of the Drawings

The invention will now be described by way of non-limiting example only, with reference to the accompanying drawings.

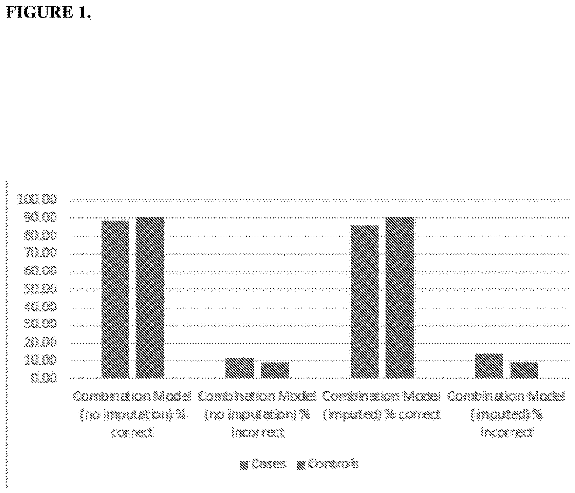

**Figure 1****.** Percentage of symptomatic (case) and symptomatic (control) participants correctly identified by MIRAT Combined Model (imputed and non-imputed).

### Detailed Description of the Invention

The articles "a" and "an" are used herein to refer to one or to more than one (*i.e.,* to at least one) of the grammatical object of the article. By way of example, "an element" means one element or more than one element.

Throughout this specification and the claims which follow, unless the context requires otherwise, the word "comprise", and variations such as "comprises" or "comprising", will be understood to imply the inclusion of a stated element, integer or step, or group of elements, integers or steps, but not the exclusion of any other element, integer or step, or group of elements, integers or steps.

As used herein "MIRAT" refers to the Mental Illness Risk Assessment Test described and exemplified herein. MIRAT may also be referred to herein as the MIRAT multi-domain model or the multi-domain model for schizophrenia and schizoaffective disorder. The term "control" or "control sample" as used herein refers to one or more biological samples from individuals or groups of individuals classified as not having schizophrenia or psychosis and where the diagnosis for the "control" or "control sample" has been confirmed. A "control sample" may comprise the compilation of data from one or more individuals whose diagnosis as a "control" for the purposes of the present invention has been confirmed. That is, for the purposes of practicing embodiments of the present invention samples to be used as controls need not be specifically or immediately obtained for the purpose of comparison with the sample(s) obtained from the subject under assessment.

The Mental Illness Risk Assessment Test is based upon a model of schizophrenia and schizo-affective disorder, composed of a number of combined biomarkers, divided into a number of functional domains, designated herein as the "MIRAT Model". This model outlines components that the inventor considers to be key ingredients of the schizophrenia, schizo-affective disorder and psychosis (including schizophrenic and schizo-affective psychosis) conditions. The model consists of five main domains (referred to herein as the 5-domain model (5DM)) and one supplementary domain of bio-neuro-sensory-cognitive markers (its inclusion giving the 6-domain model (6DM)). The first domain consists of measures of catecholamine neurotransmitter status (Neurotransmitter Domain), the second domain consists of a measure of oxidative stress (Oxidative Stress Domain), the third domain consists of measures of vitamin and mineral status (Nutrition-Biochemistry Domain), the fourth domain consists of measures of visual performance and processing (Visual Processing Domain), the fifth domain consists of measures of auditory performance and processing (Auditory Processing Domain), and the sixth and supplementary domain consists of measures of middle ear physiology and performance (Middle Ear Domain).

The Neurotransmitter Domain consists of three subcomponents these being Dopamine, Noradrenaline and Adrenaline.

The Oxidative Stress Domain consists of HPL/Creatinine and is interchangeably referred to herein as the HPL/Creatinine Domain.

The Nutrition-Biochemistry Domain consists of five subcomponents these being Serum B12, Red Cell Folate, Activated B6 (Pyridoxal-5'-phosphate coenzyme form), the ratio of Free Copper to Red Cell Zinc, and Serum Vitamin D (25-OH).

The Visual Processing Domain consists of four subcomponents these being Visual (symbol) span, Threshold visual speed of processing performance as a percentage of age (expresses threshold visual order processing speed in terms of the visual processing system's relative age), Visual speed of processing performance percentile, (expresses threshold visual order speed of processing performance as a percentile), and Distance vision predominantly on the right (binocular distance vision acuity).

The Auditory Processing Domain consists of four subcomponents these being Reverse digit span (measures auditory (verbal) working memory), competing words performance for age as a percentage of age (dichotic listening performance test, measures intra-cerebral processing of auditory information), threshold auditory speed of processing as a percentage of age (expresses threshold auditory order processing speed in terms of the auditory processing systems relative age), and auditory speed of processing performance percentile (which expresses threshold auditory order speed of processing performance as a percentile).

The Middle Ear Domain consists of six subcomponents these being Percentage length of the base of the stapes reflex divided by the total duration of the reflex (a measure of the strength of the stapes reflex during its maximal period of contraction), projected Stapes amplitude (alternative measure of stapes contraction strength), time-to-off-set of the stapes reflex contraction divided by the base length (gives a measure of any acoustic reflex offset advance or delay), threshold ear canal volume, threshold peak middle ear pressure, and threshold gradient middle ear pressure.

Scaled median absolute deviation can be used to determine proxy standard deviation for ROC cut-off values adjusted for their position in the distribution of the variable. Each subcomponent of the MIRAT Model is scored to a one or a zero using its unique cut-off value that was identified through ROC analysis. The subcomponent scores are tallied for the Domain and then the Domain is scored as a one or a zero based on its unique cut-off value that was identified through ROC analysis. Domains with missing subcomponent values but a sufficient subcomponent tally to code the Domain to one or zero are imputed. The scores for each Domain are combined (tallied) to provide a total score for the MIRAT multi-domain Model. The minimum score is zero and the maximum score is five. The combined MIRAT Model score is used to identify the risk of schizophrenia/psychosis being present and/or developing in the future. Non-parametric and Log-logistic Regression models identify the risk of schizophrenia/psychosis based on a combined MIRAT Model score of 1 through to 5. A combined score of 3 or more abnormal Domains is indicative of a significant risk of diagnosis of schizophrenia/psychosis. The Middle Ear Domain is used in borderline cases to supplement the information provided by the main combined Five Domain Model.

As exemplified herein the inventor has carried out a statistical analysis of biochemical markers, and cognitive and sensory processing measures in both the auditory and visual domains, from symptomatic schizophrenic participants and asymptomatic participants. Receiver operating characteristic (ROC) analysis identified a wide range of abnormal outcome measures across catecholamine, nutritional, auditory and visual processing and cognitive domains, confirming the heterogeneous nature of schizophrenic psychosis. These markers were combined to form a combined model (MIRAT Model) of schizophrenia/schizo-affective disorder and psychosis, which demonstrated a sensitivity of 73-93% and a specificity of 80-96% at the 95% level of significance. When odds-ratio analysis was performed (as described above), an abnormal score for three or more MIRAT model domains was associated with a diagnosis of schizophrenia, schizoaffective disorder or psychosis at 95% level of confidence and an abnormal score for four MIRAT model domains was highly associated with a diagnosis of schizophrenia or schizoaffective disorder at 95% level of confidence. When regression-analysis was performed (as described above), a diagnosis of schizophrenia, schizoaffective disorder or psychosis was predicted at 95% level of confidence for three or more abnormal MIRAT model domains, with a score of four or five abnormal domains being highly predictive of a diagnosis of schizophrenia, schizoaffective disorder or psychosis, at a 95% level of confidence.

Accordingly, the MIRAT model described herein provides a combination of biomarkers across multiple dimensions that possess diagnostic screening capacity for schizophrenia, schizo-affective disorder and psychosis, including schizophrenic and schizo-affective psychosis. A MIRAT assessment can be completed by a mental health professional, including general practitioners and nurse practitioners, within a half hour consultation time frame, in every day clinical settings with low ambient noise, using easily accessible, inexpensive equipment and simple to apply methodology. The findings described herein and the employment of the MIRAT model has the potential to form the basis for a new paradigm and new psychiatric classification system for schizophrenia, schizo-affective disorder and psychosis.

In one aspect, the present invention provides a method for diagnosing schizophrenia, schizo-affective disorder and/or psychosis in an individual or predicting risk of the individual developing schizophrenia or psychosis, the method comprising
(i) determining values for one or more markers in each of five domains in one or more biological samples obtained from the individual:
   (a) a neurotransmitter domain comprising dopamine, noradrenaline and adrenaline;
   (b) an oxidative stress domain comprising urinary hydroxyhemopyrroline-2-one and urinary creatinine or other marker of oxidative stress;
   (c) a nutrition-biochemistry domain comprising free copper to zinc ratio, activated vitamin B6, red cell folate, serum vitamin B12, and vitamin D;
   (d) a visual processing domain comprising visual span, visual speed of processing discrepancy, visual speed of processing, and distance vision on right; and
   (e) an auditory processing domain comprising reverse digit span, competing words discrepancy, auditory speed of processing discrepancy, and auditory speed of processing;
   and
(ii) comparing values for said one or markers in each of said domains to control values of said markers in subjects not suffering from schizophrenia, schizo-affective disorder or psychosis, wherein the values of said markers indicative of schizophrenia or psychosis are, relative to said control values:
   - in the neurotransmitter domain, high dopamine, high noradrenaline, and high adrenaline;
   - in the oxidative stress domain, high urinary hydroxyhemopyrroline-2-one divided by creatinine;
   - in the nutrition-biochemistry domain, high free copper to zinc ratio (or low zinc to free copper ratio), low activated vitamin B6, low red cell folate, high serum vitamin B12, and low vitamin D;
   - in the visual processing domain, low visual span, high visual speed of processing discrepancy (percentage of age), low visual speed of processing (percentile), and poor distance vision on right; and
   - in the auditory processing domain, low reverse digit span, high competing words discrepancy (as percentage of pass score), high auditory speed of processing discrepancy (as percentage of age), and low auditory speed of processing (as percentile).

Optionally, the method comprises determining values for one or more markers in a middle ear domain comprising threshold ear canal volume, threshold peak middle ear pressure, threshold gradient middle ear pressure, threshold stapes amplitude projected, threshold time to offset over baselength and threshold percentage baselength over duration, and comparing values for said one or more markers to control values of said markers in subjects not suffering from schizophrenia, schizo-affective disorder or psychosis, wherein the values of said markers indicative of schizophrenia, schizo-affective disorder or psychosis are, relative to said control values, high threshold ear canal volume, low threshold peak middle ear pressure, high threshold gradient middle ear pressure, high threshold stapes amplitude projected, low threshold time to offset over baselength and high threshold percentage baselength over duration.

Typically, the method comprises conducting statistical analysis of determined values of said markers in combination and diagnosing schizophrenia, schizo-affective disorder or psychosis is said individual on the basis of combined analysis. Typically said statistical analysis comprises receiver operating characteristic (ROC) analysis. In particular embodiments, said method comprises conducting statistical analysis of determined values of said markers in combination and diagnosing schizophrenia, schizo-affective disorder or psychosis in said individual on the basis of the combined analysis. Said statistical analysis may comprise Receiver Operating Curve (ROC) ranges for individual ROC variables and summated sets of ROC variables, using an appropriate means to determine proxy standard deviation for ROC cut-off values adjusted for their position in the distribution of the variable. Statistical analysis may also comprise using summated sets of ROC domain scores, based on cut-off values, as described above and/or odds-ratio or regression analysis of the summated ROC scores of multiple ROC domains, for the purpose of association with diagnosis or risk prediction. As exemplified herein, odds-ratio analysis was performed to evaluate the association of the number of abnormal domains with a diagnosis of schizophrenia or schizoaffective disorder and regression analysis of summated ROC scores was performed to estimate the predictive capacity of the combined MIRAT ROC model (with imputed values). A validation correlation matrix comprised of Spearman correlation coefficients (rho), shows high level correlations with summated ROC model scores and sets of ROC variables for severity, disability and treatment resistance, demonstrated significance at the 95 per cent level of significance (see Examples).

In particular embodiments, individual components of the MIRAT multi-domain model are variables identified as significant on ROC analysis. These variables are gathered together into domains of interest, the neurotransmitter domain, the nutrition-biochemistry domain, the oxidative stress domain, the visual processing domain and the auditory processing domain in which, in particular embodiments, individual ROC component results are summated to form a model under the same name. When the five principal ROC models are in turn summated, they yield the combined ROC model (imputed), which is a biomarker model for schizophrenia, schizo-affective disorder and psychosis - also called "the MIRAT model". When individual component variables within each domain are scored against their ROC cut-off point, they indicate whether or not a ROC domain itself is scored as abnormal. The score of number of abnormal ROC domain found, contributes to association with (odds-ratio) or risk of (regression-analysis), receiving a diagnosis of schizophrenia, as outlined in Tables 3 and 4 herein. The sixth domain (the middle ear domain) is an optional, supplementary domain, which can be used to increase risk prediction sensitivity in cases where prediction yields marginal or borderline results.

Typically an analysis in accordance with the present invention is carried out using each of the markers of the neurotransmitter domain, the oxidative stress domain, the nutrition-biochemistry domain, the visual domain and the auditory domain. A decision may be made by the assessing clinician as to whether or not to include the supplementary middle ear domain. For example, in cases where a diagnosis based on the neurotransmitter domain, the oxidative stress domain, the nutrition-biochemistry domain, the visual processing domain and the auditory processing domain may not be conclusive, it may be decided to proceed to middle ear testing and include the middle ear domain.

The multi-domain model (MIRAT) described herein is a novel approach collecting together biomarkers from five (and optionally six) different domains of biological, neuro-sensory and neuro-cognitive dysfunction. The domains of interest are represented by the neurotransmitter domain, the oxidative stress domain, the nutrition-biochemistry domain, the visual processing domain, the auditory processing domain, and optionally the middle ear domain. Within these domains are sub-components that are measures reaching biomarker status on ROC analysis and demonstrate an ability to inform regarding the cumulative components of schizophrenia, schizo-affective disorder and psychosis, including schizophrenic and schizo-affective psychosis, and also to both confirm association with and predict risk of the diagnosis of schizophrenia, schizo-affective disorder and psychosis.

Biochemical tests used to determine biomarker levels in accordance with embodiments disclosed herein may be carried out utilising any means known in the art and the present invention is not limited by reference to the means by which the biomarker levels are determined. Determination of biomarker levels may comprise detection and/or quantitation and the methods and techniques available for such determination are well known to those skilled in the art. Suitable methods and techniques include, but are not limited to, the use of spectral analysis, column chromatography, gel electrophoresis, mass spectroscopy and identification of protein spots, enzyme-linked immunosorbent assay (ELISA), Western blot, photonic molecular sensing techniques, image acquisition and analysis (such as magnetic resonance imaging (MRI) spectroscopy and single photon emission computed tomography (SPECT)) or other in-vivo imaging methods. Biochemical tests used to determine biomarker levels in accordance with embodiments disclosed herein may be employed in any suitable environment or setting, such as a hospital, clinic, surgical or medical practice, or pathology laboratory. Alternatively, or in addition, such biochemical tests may be incorporated into one or more devices capable of analysing the desired biomarkers to thereby allow a degree, or complete, automation of the testing process. Suitable devices are typically capable of receiving a biological sample, analysing one or more biomarker levels in said sample and providing data on said biomarker level(s) in real time thus facilitating bench-to-bedside and point-of-care analysis, diagnosis, risk assessment and/or treatment. Suitable devices include, but are not limited to, the Cobas® *in vitro* diagnostic systems (Roche Diagnostics). The device may be a handheld device or an assay device containing micro-chip technology.

Similarly, measurements of sensory parameters (including cognitive, visual, auditory and other non-biochemical markers) may be made using techniques and methodologies well known to those skilled in the art and the present invention is not limited by reference to the means by which such measurements are made.

Diagnoses and risk predictions made in accordance with embodiments disclosed herein may be correlated with or determined in conjunction with conventional diagnoses, for example as generally exemplified by the International Diagnostic and Statistical Manual of Mental Disorders, Fourth Edition, (DSM IV or DSM IV-R) or DSM V or other international mental disease or symptom classification systems known to those skilled in the art.

Accordingly, methods of the present invention may include assessment and/or monitoring in subjects of one or more symptoms associated with schizophrenia, schizo-affective disorder and/or psychosis. Without limiting the scope of the present disclosure, exemplary symptoms may include somatic concern, anxiety, depressed mood, suicidality, guilt, hostility, aggression, elated mood, grandiosity, pressure of speech, suspiciousness/persecution, auditory or visual hallucinations, ideas of reference or control, unusual or bizarre thought content, loose associations of thought, thought disorder, bizarre behaviour, self-neglect, self-harm, threats to others, disorientation, conceptual disorganisation, blunted or flat affect, emotional withdrawal, apathy, social withdrawal, social anxiety, motor retardation, tension, uncooperativeness, excitement, inattention, distractibility, motor hyperactivity, mannerisms or posturing, movement disorder, delusions, poor rapport, passivity, poor abstract thinking, reduced or absent theory of mind, reduced insight, reduced judgement, reduced short or long-term memory, anti-social traits, tendencies or acts, chronic regional pain or other unexplained chronic pain syndrome, offending behaviour of a forensic nature, disturbance of volition, poor impulse control, anger, delayed gratification difficulty, affective-lability, mood lability, mood swings, active social avoidance, preoccupation, obsessional preoccupation, ruminations, disturbance of spontaneity or flow of conversation, poor self care, anxious worrying, tension, tonicity, grasp strength, rumination, fear, active/intentional and passive/unintentional avoidance, dissociation, stress, attenuated psychotic symptoms, overvalued ideation, brief intermittent psychotic symptoms, subjective self-disturbance, re-experiencing phenomena, sense of presence, distancing, corporeality, disturbed stream of consciousness, self-other boundary disturbances, self-demarcation disturbances, body-image disturbances, anorexia, orientation and re-orientation disturbances, self-consciousness, first rank passivity symptoms, ideas of reference or control, loss of sense of self, thought insertion, thought broadcasting, thought blocking, thought replacement, abnormal perception, delusional attribution or interpretation, under-arousal, disinhibition, impulsivity, over-arousal, difficulty attending, reduced attention span, scattered attention, distressing recollections, emotional dysregulation, implausible belief, obsessional thought-preoccupation or thoughts, compensations, intrusive auditory thoughts, euphoria, apathy, and irritability or poor impulse control.

In particular embodiments the symptom(s) assessed and/or monitored in subjects may include delusions, conceptual disorganisation, hallucinatory behaviour or hallucinations (visual, auditory or olfactory), excitement, agitation, tension, grandiosity, suspiciousness, persecutory thoughts, hostility, blunted affect, emotional withdrawal, poor rapport, passivity, apathy, social withdrawal, difficulty in abstract thinking, lack of spontaneity and flow of conversation, stereotyped thinking, somatic concerns, anxiety, fear, phobia, obsessional thoughts or behaviour, mannerisms and posturing, depressed mood, depression, motor retardation, catatonia, uncooperativeness, unusual thought content, disorientation, poor attention, poor memory, lack of judgement and insight, disturbance of volition, poor impulse control, preoccupation, active social avoidance, anger, difficulty in delaying gratification, affective (emotional) lability, suicidal thoughts, suicidal intent, suicide threat or completed suicide, self-harm thoughts, self-harm deeds, bizarre behaviour, elated mood, and guilt.

One advantage offered by the present invention is the ability to reveal to the clinician objective evidence of an individual's areas of pathology and unmet needs in areas such as abnormal neurotransmitters, vitamins and minerals (nutrition-biochemistry domain), visual and auditory processing, and middle ear performance. From this position, the clinician can initiate specifically targeted remedial interventions. For example, specific remediation of competing words (dichotic listening) deficit through dichotic training may be warranted, using for example the Digit Offset Therapy (DOT) approach. Specific remediation techniques are available for correction of each of the sub-component of the MIRAT model, and will be well known to those skilled in the art. Collaborative and simultaneous implementation of these remediation techniques may assist global correction of the schizophrenia/schizo-affective condition.

Accordingly, the present invention provides methods for determining effective treatment regimes for sufferers of schizophrenia, schizo-affective disorder and/or psychosis, including schizophrenic and schizo-affective psychosis, by carrying out diagnostic tests as described herein, optionally over time and determining if there is a change over time concomitant with, or resulting from, the employment of a specific treatment regime. Also provided are methods for monitoring treatment regimes, including monitoring for treatment progress, preventing patient relapse or managing treatment resistance, by carrying out diagnostic tests as described herein over time and determining if there is a change over time concomitant with, or resulting from, the employment of a specific treatment regime. The above described methods optionally also comprise assessments and monitoring of one or more symptoms associated with schizophrenia, schizo-affective disorder and psychosis as described elsewhere herein. Thus, the present invention provides means of intervention in the treatment of a subject if necessary.

Accordingly, provided in an exemplary embodiment is a method for evaluating the efficacy of a treatment regime in a subject suffering from schizophrenia, schizo-affective disorder and/or psychosis, the method comprising:
(a) treating the subject with a treatment regime for a period sufficient to evaluate the efficacy of the regime;
(b) obtaining one or more biological samples from the subject;
(c) determining the levels of a panel of markers in the sample(s) as disclosed herein;
(d) repeating steps (b) and (c) at least once over a period of time; and
(e) determining whether the marker levels changes over the period of time.

Disease control in the subject may then be improved by adjusting the timing, frequency and/or intensity of marker testing and /or adjusting the identity, timing and/or intensity of a treatment regime to thereby normalise the levels of one or more of the markers.

The term "disease control" as used herein means the status of the schizophrenia, schizo-affective disorder or psychosis, typically in light of treatment or therapy intervention. Thus "disease control" describes the range and severity of symptoms and conditions experienced and suffered by patients as a result of their schizophrenia, schizo-affective disorder or psychosis. Disease control effectively provides a measure at a given point in time of the disease status of an individual, reflecting both current therapeutic treatment regimes used by the individual and the individual's recent experiences and psychological state.

Also provided in an exemplary embodiment is a method for designing a suitable treatment regime for a subject suffering from schizophrenia, schizo-affective disorder and/or psychosis, the method comprising monitoring the levels of a panel of markers in the subject as described herein, in the presence or absence of a treatment regime for treating the schizophrenia, schizo-affective disorder or psychosis and adjusting the identity, timing and/or intensity of the treatment regime so as to normalise the levels of one or more of the markers.

The multi-domain model (MIRAT) and test as described herein enables a clinician to quantify and understand an individual's areas of pathology and unmet needs in areas such as abnormal neurotransmitter levels, nutrition, visual and auditory processing, and middle ear performance. From this position, the clinician can become educated about the underpinnings of schizophrenia and/or schizoaffective disorder, and the underpinnings of their discreet symptoms and research evidence that already exists for specifically targeted remedial interventions in areas of unmet biological or neuro-sensory need.

The MIRAT multi-domain model has application for relapse-prevention, managing treatment-resistance and illness-prevention for schizophrenia and schizoaffective disorder. The multi-domain model and its MIRAT test may also be used to monitor clinical progress and determine treatment- response in the clinical setting. In the research setting it may be used to determine efficacy of new treatments for schizophrenia and/or schizoaffective disorder. The MIRAT multi-domain model also has application for neuro-cognitive-physiological and biological characterization of schizophrenia and schizoaffective disorder. Symptom profiles derived from biomarkers within the MIRAT multi-domain model can assist the clinician to manage problematic symptoms in a clinical setting where a MIRAT test is not available.

The following are provided, by way of example only, as means of employing the MIRAT model and its domains and their component ROC variables in relationship to key symptoms and behaviours associated with schizophrenia, schizo-affective disorder and psychosis:
(1) There is a general need for knowledge about the underpinnings of classical psychiatric symptoms and behaviours and the MIRAT domainsmay serve as endophenotypes for schizophrenia. The MIRAT model itself has the potential to serve as a nidus or template for a new classification system for serious mental illness symptoms and behaviours.
(2) It may also be that in some clinical settings such as rural and regional areas where full MIRAT model testing cannot be conducted, clinicians could still benefit from research-based-evidence regarding specific substrates of a particular problematic behaviour and/or symptoms. Understanding these behaviours and/or symptoms in terms of their biological, nutritional and neurosensory/cognitive correlates, allows clinicians a broader range of responses in their management of such conditions in the clinical setting. For instance, the knowledge that paranoid aggression and/or hostility correlates closely with certain nutritional and neuro-sensory and neuro-cognitive abnormalities, may assist to initiate urgent interventions that together with pharmacotherapy allows remediation of aggression to a sub-threshold level, offsetting risks associated with management or containment. e.g. if it is known that an aggressive, hostile, paranoid patient is likely to have nutritional and auditory processing problems, nutritional replacement, ward milieu adjustment, adjusted staff communication style and in the longer-term specifically targeted intervention, may assist to offset the severity of this behaviour. A further instance is the knowledge that suicidality, suicidal behaviour or intent may be associated with certain nutritional, neurotransmitter and neuro-sensory-cognitive abnormalities, may inform and assist prevention of suicide.

The present disclosure contemplates any suitable means of employing the MIRAT multi-domain model testing. For example, the model may be suitably employed via a computerised system, including an online, internet-based platform or via an app suitable for a computer or personal electronic device such as a tablet, smartphone or other PDA or mobile device. Such an app may be developed for use on any operating system, including for example the Apple iOS and Android operating systems.

Embodiments disclosed herein also contemplate the use of one or more additional biomarkers to aid in diagnosis and risk predictions. Such additional biomarkers may, for example, be used to validate or extend diagnoses made in accordance with the present disclosure. Such additional markers may be markers of, for example, inflammation, tissue damage, oxidative stress, urine excretion function and histamine metabolism. Suitable 'validation' markers may include, for example, 1- methyl histamine, histamine, histidine, S-adenosyl-methionine (SAMe), S-adenosyl homocysteine (SAH), ratios between S-adenosyl-methionine and S-adenosyl homocysteine, serum/plasma adenosine, reduced and oxidised glutathione and ratios between reduced and oxidised glutathione, vitamin B2 (riboflavin) and associated molecules such as flavin adenine dinucleotide (FAD), flavin mononucleotide (FMN), F 450, L-threonine, quinone, semiquinone and flavin synthase, urine or plasma L biopterin, tetrahydro-L-Biopterin,(BH4) 7,8-dihydro-L-Biopterin (BH2) , BH4:BH2 ratio 5-hydroxy indole acetic acid, platelet monoamine oxidase, red cell N-methyl transferase, catechol-O-methyltransferase polymorphisms, methyl tetrahydrofolate reductase polymorphisms (C667T and A1298C forms), thyroid stimulating hormone, serine, glycine, thromboxane, urine homovallinate, vanilmandelic acid, serum creatinine, immunoglobulins A, E, G, & I., IgG and IgE food allergy screens, IGE allergy correlates, all inflammatory cytokine levels, TNF alpha and interferon kappa B, C reactive protein, serum iron (ferritin, transferrin, transferrin saturation), serum, plasma or urinary lead, antigliadin antibodies, red cell/serum magnesium, serum calcium, free calcium concentration, blood sugar and plasma insulin, N-acetylaspartate, D glucaric acid, phosphocreatinine, glutamate dehydrogenase, N methionine adenosyl transferase, plasma retinol, B-retinylacetate, B-retinoic acid, tyrosine hydroxylase, thyroxine T3, T4 and reverse T3 components, serum creatinine, prostoglandin El, catalase (CAT), reduced glutathione (GSH), oxidised glutathione (GSSG), antioxidant ratio (GSH/GSSG), vitamin C, albumin, nicatimamide adenine dinucleotide (NADPH oxidase) deficit, glutamate-cysteine ligase (GCL and GCLC), glycerol dehyde-3- phosphate (GAPDH), haeme oxygenase (HO-1), 3-nitrotyrosine (3NT), 8 oxo-deoxyguanosine (8-oxo-dG), 3 chlorotyrosine (3-CT), aconitase activity, H2DCFDA (DCF), advanced glycation end product (CML) or lipid peroxidase marker (HNE).

Further additional markers that may be measured or assessed in conjunction with the markers hereinbefore disclosed and in accordance with embodiments disclosed herein include, but are not limited to: urinary porphyrins including total urinary haeme, urinary precoproporphyrin (COPRO), keto-isococoporphyrin, urinary uroporphyrin (URO), urinary precoproporphyrin (PRECOPRO), PRECOPRO:URO ratio, uroporphyrin decarboxylase (UROD), cocoporphyrinogen oxidase (CPOX), hepta and hexacarboxyporphyrins, 5-aminolevulinic acid (gamma ALA), urinary coproporphyrinogen and faecal isococproporphyrin); serum/plasma 1 methyl histamine; tGSH:GSSG ratio; glutathione peroxidase; superoxide dismutase; glutathione S transferase P1 (GST P1); glutathion-S-transferase M1 (GST M1); urinary alphahydroxybutyrate; urinary DHPG : MHPG ratio; urinary pyroglutamate; urinary sulphate; urinary 8-hydroxy-2-deoxyguanosine; red cell folic acid; red cell methyl malonic acid; urinary forminoglutamate; serum/plasma adenosine; red cell pyridoxine activation test; red cell transketolase; red cell pyridoxal phosphate activation test; plasma cysteine; total glutathione (reduced) glutathione (GSSG); urinary or plasma tetrahydrobiopterin BH4; red cell pyridoxine activation test; red cell transketolase; urinary xanthurenate; urinary kynurenate; 25 hydroxy cholecalciferol; vitamin D receptor polymorphisms; urinary DOPAC: HVA ratio; vitamins CoQ10, E, A, or D; urinary adipate; urinary suberate; urinary ethylmalonate; APOE polymorphisms; urinary methylmalonate; serum/plasma methionine; serum/plasma S adenosyl methionine; red cell magnesium; serum magnesium; serum Fe 10; ferritin; transferrin; serum cortisol; DHEAS; urine imidazole acetic acid, whole blood histamine, substance P; urinary alpha-ketoisovalerate; urinary alpha-ketoisocaproate; urinary alpha-keto-b-methylvalerate; urinary beta-hydroxyisovalerate; urinary HIAA (5-hydroxyindoleacetic acid); urinary DOPAC (3 -methoxytyramine); histidine methyl transferase, urinary HVA (homovallinate); urinary DHPG (dihydroxyphenylglycol); urinary MHPG (urinary 3-methoxy-4-hydroxyphenylglycol); urinary DOMA:VMA; red cell catechol-o-methyl transferase (COMT) including polymorphisms; MRNA for 7 nicotinic acetylcholine receptor, choline creatinine ratio, phosphocreatinine, alpha C-methyl-L-tryptophan trapping, N acetyl aspartate, eosinophil protein X and eosinophil calprolectin, plasma S adenosyl homocysteine; S adenosyl homocysteine hydrolase; platelet catecholamines; urinary hydroxymethylglutarate; blood lymphocyte 7 nicotinic acetylcholine receptor, IGG food allergy screen; imidazole N-methyl transferase., B2 microglobulin; antigliaden autoantibodies (such as tissue transglutamase IGG, tissue transglutaminase IGA, Methionine adenyl transferase, endomysial antibody); urinary p-hydroxyphenylacetate; CD8 and SD4 T cell levels; inflammatory cytokine levels; urine methyl histamine, urine histamine, C reactive protein; erythrocyte or serum N methyl transferase, nerve growth factor; arginine N methyltransferase; urinary VMA (vanilmandelic acid); vesicular monoamine trasnporter (VMAT2); neuronal nitric oxide synthetase; alpha-C methyl-L-tryptophan; acetyl cholinesterase, choline acetyltransferase; vesicular acetylcholine transporter; and tyrosine hydroxylase; red blood cell choline, alpha 7Acetylcholine receptor activity, alpha 4 acetylcholine receptor activity, acetylcholine esterase, glutamic acid decarboxylase, taurine, adenosine, kainate, glycine, spermine, spermidine, glutamate, substance P, aspartate, biotin,, quinolones, quinolinate, quinolinic acid, picolinate, kynurenic acid, free androgen index, urinary phydroxyphenyl acetate, serum histidine, urinary DOMA (3,4-dihydroxymandelic acid); plasma nitrous oxide; Cu:Zn ratio (N 0.8-1.2); free copper (Cu); urine histamine; plasma chromium; whole blood serum and urine lead (Pb), mercury (Hg) and cadmium (Cd); hair mineral analysis for cadmium, mercury, arsenic, lead, copper, chromium, lithium, sodium, potassium, bismuth and chloride; urine whole blood, red cell and/or serum assays of vitamin A, plasma methyl-malonic acid; plasma, blood and/or urine assay of pyridoxine-5-phosphate (P5P), pyridoxil kinase, niacin, niacinamide, red cell transketolase; thyroid stimulating hormone; thyroid peroxidase antibodies; free T3 and T4; reverse T3; serum cortisol; urinary iodine, urinary folate as urinary fromino-glutamic acid (FIGLU); urinary N-methyl- Nicotinamide.methylmalonic acid; erythrocyte glutamic-pyruvic transaminase (EPGPT); glutamic-oxaloacetic transaminase (EGOT); serum levels of electrolytes, Ca⁺⁺, Mg⁺⁺ and BSL; ferritin; biopterin; C- reactive protein (CRP); serum and/or red blood cell assay of manganese; secretory IGA; serum IGA, IGG, IGM and IGE; IGG and IGE for gluten and casein sensitivity; red cell fatty acids; arachadonic acid (AA):EPA ratio; lipid peroxidises; H₂O₂; t-butylhydroperoxide; cumene hydroperoxide; 2-thiobarbituric acid reactive substances (TBARS); apometallathionein; glutamic decarboxylase; oxidative stress biomarkers including 8 hydroxydeoxyguanosine (8-OhdG), malondialdehyde (MDA) and isoprostane; glutathuione peroxidase (GSH-Px); superoxide dismutase (SOD); urine lipid peroxides; hydroxy catechol markers; glutathione transferase; S adenosylhomocysteine hydrolase; spinal motor neuron survival gene (SMN); red cell and/or serum methionine adenosyltransferase; S-adenosyl-L-methionine synthetase; methionine breath test; adenosine deaminase; urinary indicans; valerate isobutyrate; urine analysis of lactulose, mannitol and lactulose:manitol ratio after lactulose mannitol challenge; serum cholesterol; triglycerides; uric acid; serum iron; ferritin, transferrin and transferrin saturation; aspartate amino transferase (ALT), alanine amino transferase (AST); lactic dehydrogenase (LDH); low density lipoprotein (LDL); tissue transglutaminase IgG; tissue transaminase IgA; endomysial antibody; calprotectin and eosinophil protein X; interleukin IB; serum testosterone; free androgen index; DHEAS (dehydroepiandosterone); antigliadin IgA; serum transglutaminase IgA antibody; gliadin IgG antibody; full blood count; haemoglobin; faecal PH; cholesterol; pancreatic elastase; n butyrate; acetate; propionate; faecal total short chain fatty acids; total long chain fatty acids; faecal microbiology, mycology and parasitology; glycine:glucuronide ratio; sulphate:glucuronide ratio; D glucaric acid; glutamate dehydrogenase; urinary amino acids such as histidine, isoleucine, leucine, lysine, methionine, phenylalanine, threonine, tryptophan, valine, cysteine, glutamine, taurine, tyrosine, alanine, arginine, aspartic acid, glutamic acid, glycine, proline, serine, aspartate, asparagine, tyrosine, glutamine and glutamate; copper/zinc superdioxide and catalase activity; ESR; IL-1B (interleukin 1B); tumour necrosis factor alpha (TNFalpha) and serum alpha1, alpha2 and gamma fractions; platelet glutamate levels; serum holotranscobalamin; adenosylcobalamin, NMDA receptor NR2B subunits and other sub unit receptor activity; blood trype; prostoglandin E1; brain derived neurotrophic factor Val/Met polymorphisms; 5HTT-LPR polymorphisms; thiamine; omega 3; omega 6; retinoic acid; bexoratene; UA B30; blood diamine oxidase activity; blood or urine urea; blood or serum thyroxine transthyretin, thromboxane, blood or urine ammonia concentration; urinary amino-n-butyric acid; for amino glutamic acid; urine anserine; urine sarcosine; alpha-ketocaproic acid; beta aminoisobutyric acid.urine glutamic acid; glutaric acid; and glutamine/glutamate ratio; pyroglutamic acid; 3 -hydroxypropionic acid. dihidroxyphenylpropionic acid; urine arginine/ornithine ratio; citruline; kynurenic acid; serine; tyrosine; 3 methoxy-4-oh-phenylglycol; taurine; 4-hydroxyphenylpyruvic acid; suberic acid; pyruvic acid; 5 hydroxyphenylpyruvic acid; citric acid; cisaconitic acid; citric acid; aspartic acid; lactic acid; adipic acid; phenyl acetic acid; 5-hydroxy indolacetic acid; dihydroxyphenylpropionic acid; 2-hydroxyphenylacetic acid; cysteine homogentisic acid; benzoic/hippuric acid ratio; lipid peroxidases; carnosine; alpha- amino-n- butyric acid; alpha ketovaleric acid; alphaketomethylvaleric acid; alpha ketovaleric acid.; succinic acid; urine beta aminoisobutyric acid; gamma-aminoisobutyric acid; indoleacetic acid; phenylacetic acid; arabinose; malic acid; homogentistic acid; urine methylmalonic acid; urine homocysteine; urine 1-methyl histidine; 3-methyl histidine. urine succinyl purine; inosine; adenosylcobalamine coenzyme; proline; phosphoserine; ethanolamine; urine phosphoserine; urine cystathione; histidine decarboxylase (HDC), histamine-N-Methyl transferase (HNMT), monoamine oxidase A, phosphoethanolamine; orotic acid; urine n methylglycine; urine opiate peptides; IgG and IgE anti casein and gluten antibodies; plasma serine; plasma glycine; serine hydroxymethyltransferase; C¹⁴ or C¹¹ labelled CO₂ following C¹⁴- or C¹¹-methionine administration; histamine N methyl transferase (HMT); serum/plasma glycine; methylcytosine binding protein (MeCP2); histone(H4) deacetylase; acetylated histone(H4); plasma pyridoxyl phosphate; glutathione-S-transferase; cystathione beta synthetase (CBS, CbetaS); cysteine beta synthetase (CBS), S adenosyl homocysteine hydrolase (SAHH), serine hydroxymethytransferase (SHMT), sulphite oxidase, plasma alkalinepyridoxine phosphate phosphatise; mitogen phytohemagglutin (PHA); serum histamine(2-(4 - Imidazolyl)-ethylamine); red blood cell histamine; erythrocyte histamine-N-methyltransferase; glycine-N-methyltransferase; retinol binding globulin; glutathione- S-transferase; choline acetyl transferase: to acetylcholine esterase ratio, betaine homocysteine methyl transferase (BHMT), 5 methylhydrpofolate-homocysteine S methyltransferase, methionine synthetase (MS), methionine synthetase reductase, tryptophan hydroxylase, tyrosine hydroxylase, urine dimethyltryptamine (DMT); fasting blood alanine; blood lactate:pyruvate ratio; blood acetyl-carnitine: free carnitine ratio; beta casomorphin-7; casomorphin; influenza titre; glutamic acid decarboxylase 65 & 67 KDA; indoleamine 2,3,-dioxygenase (IDO), tryptophan 2,3-dioxygenase (TDO), reelin proteins; plasma rennin; serine hydroxymethyltransferase; glutathione synthetase; heart rate; blood pressure; continuous task performance; saliva cortisol; catecholamines (noradrenalin and adrenalin and metabolites); corticotrophin releasing factor; coeliac screen, urine propionate, acetate, faecal PH; cholesterol; pancreatic elastase; n-butyrate; acetate; propionate; faecal total short chain fatty acids; total long chain fatty acids; faecal microbiology, mycology and parasitology, hippurate. benzoate, faecal micro-organsim aerobe and anaerobe DNA and mRNA analysis, Glutathione synthetase, glutathione peroxidase, superoxide dismutase (SOD), glutathione-S-transferase P1, glutathione-S-tranferase M1, thiobarbituric reactive substances (TBARS), nitric oxide, glutathione peroxidise (GP), copper/zinc superoxide dismutase (SOD), copper/zinc superoxide dismutase, sulphate to sulphide ratio, lipid peroxidases, urine or other bodily fluid ammonia level/concentration, blood ammonia and expired or other ammonia levels , sulphite oxidase, lymphocyte DNA methylation, 8 hydroxy-deguanosine, hydroxyl-2-dehydroxyguanosine, C reactive protein, orotate, tricarb valerate, Kynurenate: quinolinate ratio, glutamate:semiquinolone ratio, dimethyltryptamine L tryptophan, glutamate/dopamine ratio, noradrenaline: substance P ratio, plasma or urinary formate or formic acid, kynurenate/kynurenic acid ratio, lactoferrin,, urinary alphahydroxybutyrate, urinary sulphite to sulphate ratio, red blood cell Catechol-o-methyltransferase activity, histamine-n-methyltransferase activity. 3 hydroxy kynurenine, xanthurenic acid, cystathione, neopterin, arginine:citrulline ratio, plasma oxytocin, thioredoxin (TRX), alanine amino transferase (ALT), gamma-aminobutyric acid (GABA), parvalbumin immunoreactivity.

For markers listed above, measurements may be made of levels, ratios and/or activities, affinity, radioligand binding levels, or other means of biomarker or receptor activation assessment, subunit messenger RNA expression and levels as appropriate. For enzymes, measurements may be of levels, activity, V max and/or Km, kcat, kcat/Km. For genes listed, measurement may be of single nucleotide polymorphisms and isomers, sequence deletions, inclusions, repetitions, isomers, missense mutations, micro DNA or abnormalities of specific interest.

Biological samples used to determine levels of any biochemical markers contemplated herein may be derived from any suitable body fluid or tissue. For example the sample may comprise blood (such as erythrocytes, leukocytes, whole blood, blood plasma or blood serum), saliva, sputum, urine, breath, condensed breath, amniotic fluid, cerebrospinal fluid or tissue (post-mortem or living, fresh or frozen). In a particular embodiment the sample comprises whole blood, blood serum or urine. In specific embodiments of the present invention the markers in the neurotransmitter domain and the nutrition-biochemistry domain are typically determined from blood or urine samples obtained from an individual to be assessed, more typically from blood samples. The markers in the oxidative stress domain are typically determined from urine samples obtained from an individual to be assessed.

In diagnosing schizophrenia, schizo-affective disorder and psychosis, including schizophrenic and schizo-affective psychosis, and predicting association with schizophrenia or risk of an individual developing schizophrenia, schizo-affective disorder or psychosis, including schizophrenic and schizo-affective psychosis, in accordance with embodiments disclosed herein, determination of markers as disclosed herein may be used in conjunction with a range of other sensory-based, cognitive and behavioural tests known and available to those skilled in the art including, for example, Go-NO-GO test, digit-symbol processing speed and accuracy test, an acoustic reflex and reflex decay test; anxiety potentiated startle reflex; startle reaction time; acoustic startle (threshold, inhibition and affective inhibition); auditory brain stem responses (ABR) such as stimulus threshold, waveform morphologies, absolute and relative amplitudes, latencies, middle latency response (MLR) and relative interpeak latencies for ABR waves N1, Na, Pa, Pb and late latency response (LLR), N1, P2 and P3 (P300) components, auditory tone (pitch) discrimination test, division of auditory attention test, filtered word test, auditory figure ground test, visual field evoked response test, prepulse inhibition test, quantitative EEG and topographic mapping of alpha, beta, theta and delta waves and all possible power ratios between these waves, including absolute power, relative power and power relative to normal data base, spectral analysis, independent component analysis, Z score analysis and signal source analysis; visual response search score; eye blink rate; mismatch negativity; auditory (and visual) evoked response potentials and the P 50, N1, P1, N2, P200, P250 and P300 components of the evoked response and their amplitude laterality discrepancies and interpeak latencies, retrograde memory; immediate memory; memory selection; executive function; N-back test; response speed; directed ignoring task; go/no go response inhibition; internal/external locus of control; strength of memory score; memory tests (e.g. Ray copy/recall, RAVLT and RAVLT errors., SILS, quick T, IT); saccadic eye movements; antisaccade task; EEG gamma band synchrony; and auditory (and visual) evoked response tests, components including mismatch negativity component (MMN), N1, P50, P400, P3a and P3b components during a cognitive task, contingent negative variation component (CNV) and post-imperative negative variation (PINV) component Auditory Brain stem Response (ABR) stimulus threshold, waveform morphologies, absolute and relative amplitudes, latencies, middle latency response (MLR) and relative interpeak latencies for ABR waves N1, Na, Pa, Pb and late latency response (LLR), N1, P2 and P3 (P300) components, ABR frequency and amplitude laterality differences, ABR interpeak latencies, frequency and power analysis of BOLD fMRI signal for sensory, motor, cognitive or integrated tasks and/or brain networks.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

The present embodiments are to be considered in all respects as illustrative and not restrictive.

The present invention will now be described with reference to the following specific examples, which should not be construed as in any way limiting the scope of the invention.

### Examples

### Example 1 - Assessment methodology

### Subject recruitment

Ethics permission for the study was obtained from the Queen Elizabeth Hospital Ethics Committee. Data from an earlier pilot study indicated that a minimum of 60 cases was needed to ensure a minimum level of significance of 90 per cent. Symptomatic participants (67 cases) were recruited from ward and community settings. Diagnoses were made by DSM IV-R criteria and were verified by the DSMIV-R symptom-checklist. Pharmacotherapy of symptomatic participants remained stable during the assessment period. Persons medicated with Clozapine, Olanzapine, or anti-histamines or vitamins were excluded. Persons with substance abuse, upper respiratory tract infections, intellectual, visual or auditory disability or documented history of head injury or extrapyramidal or motor abnormality of ocular, forearm or hand muscle movements were also excluded.

Asymptomatic mentally healthy control participants (67) were collaterally randomly selected and recruited with the assistance of the Population Research and Outcomes Studies (PROS) Unit of the University of Adelaide, via the Queen Elizabeth Hospital North West Adelaide Cohort (Health Study). In order to obtain younger controls for age and sex matching, a small number of volunteers were recruited from local surf life-saving clubs. Volunteer persons with a history of mental illness, substance abuse, or visual or hearing disability, learning disability, or taking anti-histamine medication or vitamin supplementation were excluded from selection.

### Specimen collection and biochemical assays

### Neurotransmitters

Compromised cooperation in psychotic participants precluded collection of 24 hour specimens, therefore overnight-rested and fasted participants self-collected 50 millilitres of urine (minimum of 2 hours separation from blood collection) for biogenic-amine analysis of dopamine, noradrenaline, and adrenaline. Dopamine, noradrenaline and adrenaline were measured by SA Pathology using known, routine methods. Pathology using a fasting and predominantly second void morning spot urine sample. Urine was snap-frozen to minus 30 degrees and batch transported weekly to the laboratory. Biogenic amines were analysed by mass spectrometry, using urinary creatinine as a standard, with results presented as nmol/mmol of creatinine.

### Creatinine

Creatinine in mmol/L was measured by SA Pathology using a spot urine specimen from the same void as the sample provided for HPL and urinary catecholamine assay.

### Urinary hydroxyhemopyrroline-2-one (HPL)

Urinary hydroxyhemopyrroline-2-one (HPL) analysis was conducted by Applied Analytical Laboratories. Predominantly second void specimens were collected into vials containing ascorbic acid as a preservative. These were snap frozen (-30°C) and protected from direct light until quantitative analysis was undertaken by colorimetric method at 540nm, following solvent extraction and reaction with Erich's reagent.

### Vitamins and minerals

All vitamin and mineral blood samples were fasting morning blood taken at a period separated from urine collection by at least one hour. Vitamin D (25 OH form) was measured by Clinpath laboratories using the Diasorin Liason assay method. Serum B12 was measured by Clinpath laboratories using the Roche Modular Immunoassay and reported as pmol/L. Red Cell Folate was measured by Clinpath laboratories using the Roche Modular Immunoassay and reported as nmol/L. Pyridoxal-5'-phosphate (vitamin B6 coenzyme form) was measured and reported in whole blood using High Pressure Liquid Chromatography by Sullivan Nicolaides Pathology on behalf of Clinpath laboratories, and reported in nmol/L. Serum Copper was measured by Douglass Hanly Moir Pathology on behalf of Clinpath laboratories using atomic absorption method and reported in umol/L. Red Cell Zinc was measured by Sullivan Nicolaides Pathology on behalf of Clinpath laboratories by fluorometric method on a Helena Lab instrument, and reported in umol/L. Ceruplasmin was measured by Douglass Hanly Moir Pathology on behalf of Clinpath laboratories using the Siemens IMMULITE® 2000 immunoassay system, and reported in g/L. The percentage of free copper in the serum was calculated by an equation based on the molecular and atomic weights of ceruloplasmin and copper (one ceruloplasmin molecule binds to six copper atoms). The ratio of the percentage free copper to red cell zinc was calculated as "Percentage free copper" / "Red cell zinc umol/L" .

### Sensory processing assessment

Sensory processing assessors were blind to laboratory results but unavoidably aware of residual symptoms of psychosis displayed by some patients during test-procedures.

### Visual assessment

Visual outcome measures were obtained for visual acuity, attention span, speed and accuracy of visual processing. Visual assessment was conducted using the participant's usual glasses (when applicable). Alternate-cover-test was conducted to exclude visual fixation disparity (phoria).

### Visual span

Visual-spatial attention was assessed using The Visual Symbol Test, a subset test of WMS-IV (Weschler Memory Scale) reported as the absolute number of visual symbols directly replicated in the correct order.

### Distance vision

The Snellen-Chart was used to assess distance vision on the left and the right of test subjects. Only distance vision on the right yielded a statistically significant result.

### Visual speed of processing performance as a percentage of age and Visual speed of processing performance percentile

Visual speed of processing was assessed using the Brain Boy Universal Professional instrument (MediTECH Electronic GmbH). The Brain Boy Universal Professional BrainB-v (Order-v) test measures visual speed-of-response for correctly identifying the first appearance of two visual stimuli (flashes) that are randomly presented from left-to-right or right-to-left on multiple occasions, until a threshold response speed is determined, expressed as the inter-stimulus interval (ISI) in msecs. The term "threshold" used herein refers to the lowest ISI level at which correct visual order processing (best performance) can be obtained. A read out of the threshold speed of visual (order) processing is then provided, as is a performance-age rating, which has been configured against norms-for-age in the stored data-base. For adults between the range of 18 and 60 years, the normal range for visual speed of (order) processing is 24 to 72 milliseconds.

The MIRAT multi-domain model variable, termed the *Visual speed of processing performance as a percentage of age* was calculated by subtracting the norm-for-age from the performance-age provided, which was then divided by the age of the test subject, and then multiplied by one hundred.

### Auditory processing assessment

Auditory outcome measures were of conduction, acuity, attention, threshold speed & accuracy of auditory processing. Assessments were conducted in a quiet room. Preliminary examination of the external-auditory-meatus, excluded obvious tympanic-pathology and sebum-obstruction. Audiometry examination identified undetected early-deficits in auditory-processing, as determined by measuring air-bone conduction-gap at threshold or neurosensory-deficits in the speech and language range (1000 to 4000Hz) on the MAICO Audiogram MA 40 (MAICO Diagnostic GmbH).

### Reverse digit span

Reverse digit span is a subset of the Wechsler Adult Intelligence Scale-III (WAIS-III), 1997 that is delivered verbally and measures auditory (verbal) working memory. The MIRAT variable Reverse digit span was reported as the absolute number of digits correctly recalled in reverse order.

### Competing words (performance for age as a percentage of age)

The SCAN-3 auditory screening test for adults was used to assess intra-cerebral processing of auditory information. The SCAN-3 is a validated, standardised, screening test using a voice-over CD and earphones to present the brain with several types of auditory challenge. One challenge comprises a dichotic-listening test that assesses ability to correctly identify both of two competing-words (CW), delivered separately to the right and left ears. Using this test's normative-for-age database, the difference between each test subject's expected and actual performance-for-age was calculated, and this was then divided by the age of the test subject, and then multiplied by one hundred, to create the MIRAT variable *Competing words (performance for age as a percentage of age).*

### Auditory speed of processing performance as a percentage of age and Auditory speed of processing performance percentile

The Brain Boy Universal Professional BrainB-a (Order-a) test measures auditory speed-of-response for correctly identifying the first side of hearing two auditory stimuli (clicks) that are randomly presented from left-to-right or right-to-left on multiple occasions through headphones, until a threshold response speed is determined, expressed as the inter-stimulus interval (ISI) in msecs. The term "threshold" used herein refers to the lowest ISI level at which correct auditory order processing (best performance) can be obtained.

A read out of the threshold speed of auditory (order) processing is then provided, as is a performance-age rating, which has been configured against norms-for-age in the stored database. For adults between the range of 18 and 60 years, the normal range for auditory (order) speed of processing is 46 to 72 milliseconds.

The MIRAT multi-domain model variable, termed *Auditory speed of processing performance as a percentage of age* was calculated by subtracting the norm-for-age from the performance-age provided, which was then divided by the age of the test subject, and then multiplied by one hundred. The MIRAT variable termed the *Auditory speed of processing performance percentile* was calculated from the norm age reference/performance ranges for the Brain Boy Universal Professional.

### Measures of middle ear compliance and conductance involving tympanic and stapes muscle-contraction

Ear canal volume at threshold auditory response, peak middle ear pressure at threshold auditory response, and the gradient of the middle ear pressure was measured with the Auto Tymp GSI 38 by VIASYS Healthcare. This instrument measures the parameters of the acoustic reflexes, consisting of the tympanic muscle reflex response to sound entering the ear, along with the strength and duration of stapes muscle reflex which dampens the middle-ear-conducted auditory signal entering the cochlear. In relationship to these middle ear acoustic reflexes, the term "threshold" refers to the first reflex response which typically occurs at a frequency of 500Hz in a decibel range of 90 to 110. (There was no significant differences in decibel or Hz threshold response ranges between cases and controls).

### Percentage length of the base of the stapes reflex divided by the total duration of the reflex

The GSI 38 traces the stapes reflex contraction to maximum amplitude of 8 millimetres, after which it traces a basal threshold formed for the maximum portion of the reflex. The length of this basal portion divided by the total duration of the reflex contraction, expressed as a percentage, was a measure of the strength of the stapes reflex during its maximal period of contraction.

### Stapes amplitude (projected)

Stapes amplitude projected is an alternative measure of stapes contraction strength obtained by measuring stapes amplitude at the intersection of projected onset and offset contraction gradients.

### Time-to-off-set of the stapes reflex contraction divided by the base length

The base length as described above was compared with the total duration of the stapes reflex, from its initiation to its time of offset. This gave a measure of any stapes acoustic reflex offset advance or delay in applying the handle of the stapes to the window of the cochlea.

### Severity and disability outcome measures

Participants were rated on the Clinical Global Impression of Severity (CGI), Global assessment of Function (GAF), and Social and Occupational Functioning Assessment Scale (SOFAS). Number of readmissions of each patient was taken as a measure of treatment resistance. The Brief Psychiatric Symptom Scale (BPRS), has many symptom-overlaps with those of the Positive and Negative Symptom Scale for schizophrenia (PANSS), and these two scores were amalgamated in the interest of reducing participant assessment time. The BPRS provides intensity sub-scores (1-7) for rating each symptom. The rating level for each symptom was summated to give a symptom-intensity-rating (SIR) index for each participant. Symptomatic patient-participants and asymptomatic control-participants were rated for symptoms using these measures. Ratings were collected by a single researcher, who was blind to laboratory results at the time of testing.

### Statistical analysis

Statistical analysis was complicated by the lack of normal distribution in the variables that necessitated the use of non-parametric methods of analysis and data modelling. Scaled median absolute deviation (sMAD) was used to determine proxy standard deviation for ROC cut-off values.

Table 1 summarises the principle characteristics of the variables composing the MIRAT model.

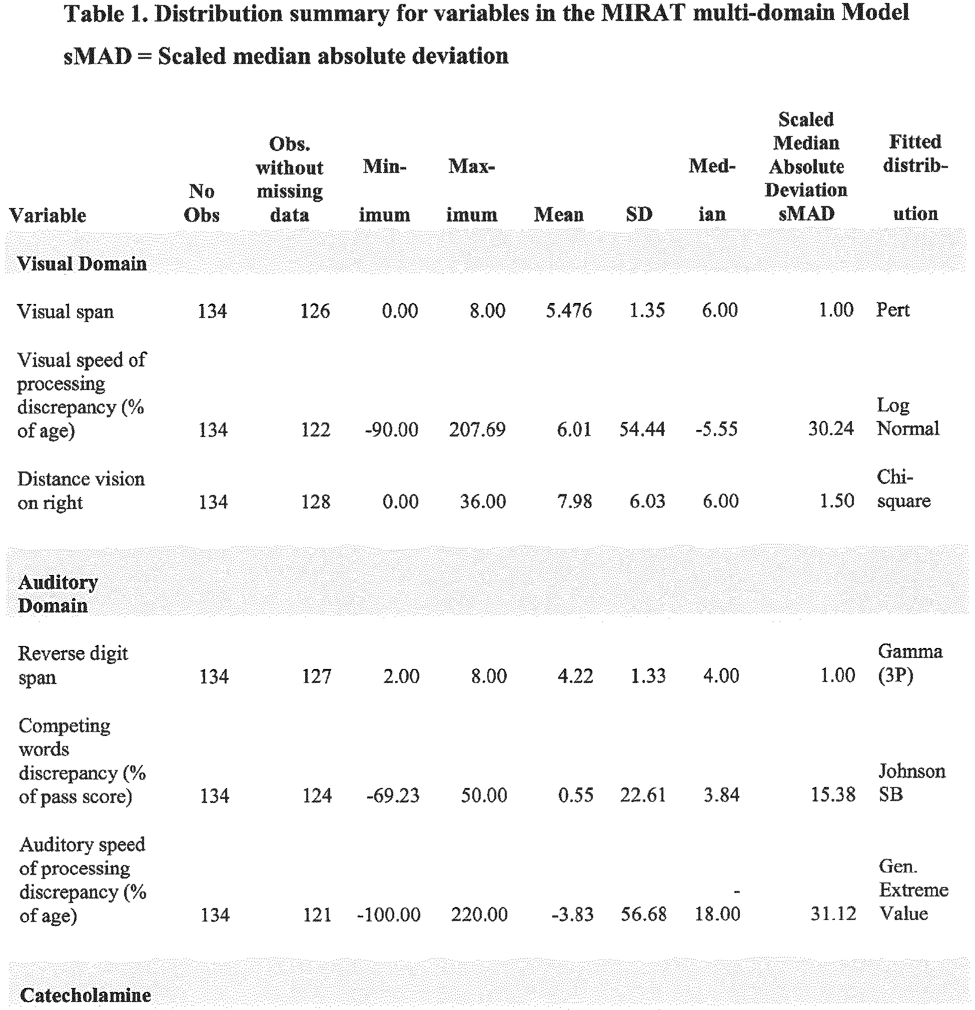

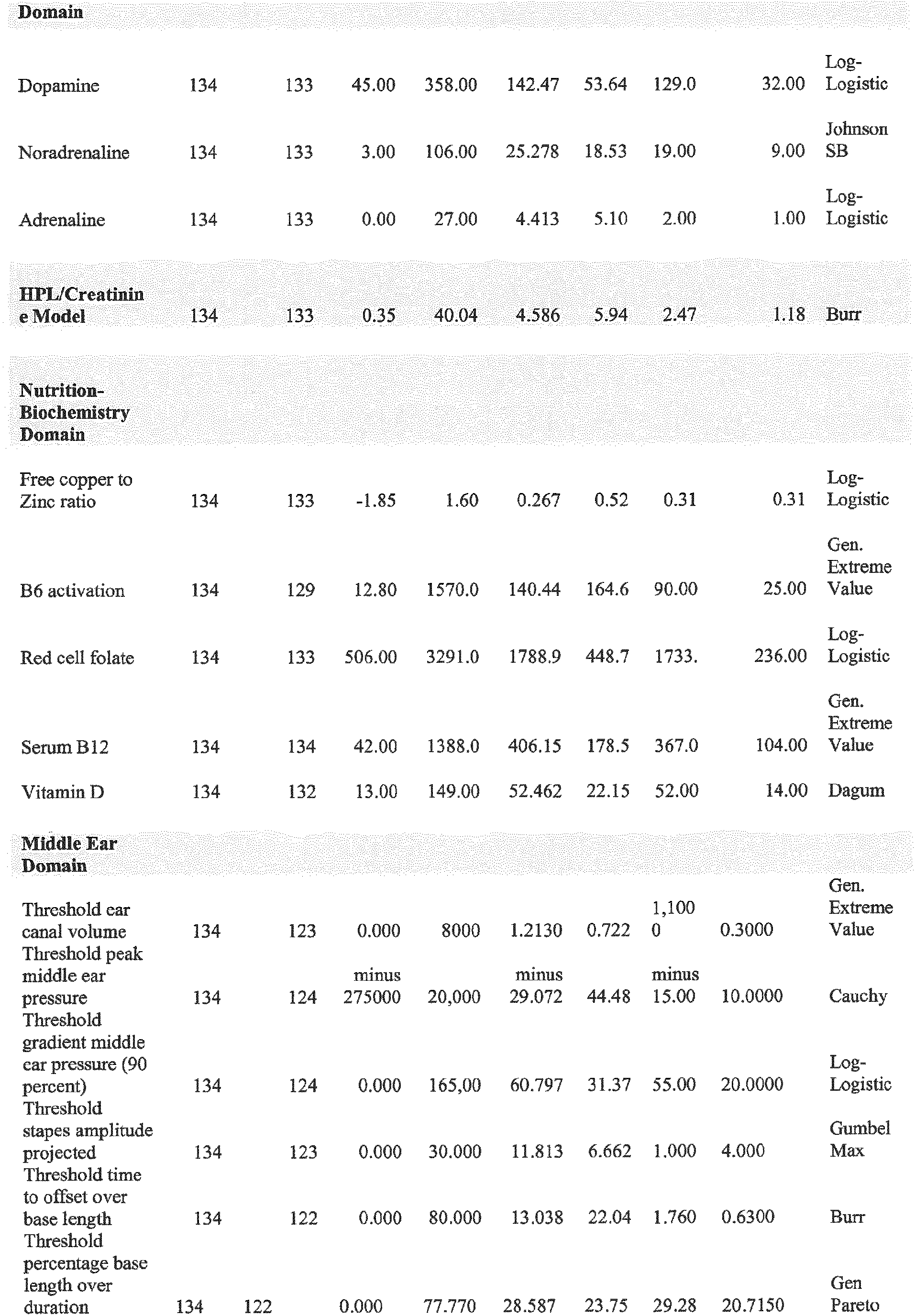

Statistical analysis was conducted using XLSTAT (Addinsoft) for descriptive statistics, ROC analysis, Sensitivity and Specificity analysis, calculation of positive and negative predictive value and likelihood ratios, Spearman's correlation, and logistic and non-parametric regression. Variable distributions were mapped using EasyFit software (Mathwave). Only variables that had a high area under the curve (AUC) or contributed to raising the AUC of a group of biomarkers were included in the MIRAT model. ROC analysis plots the sensitivity and specificity of the test result against each outcome-measurement, to give an indication of a test variable's screening and/or diagnostic utility. A cut-off point in a continuously distributed measurement delineates a normal from an abnormal result. Indices of sensitivity, specificity, positive and negative predictive value, and likelihood-ratio are also supplied. In this setting, a high sensitivity and PPV means that a test only rarely misses classifying a sick person as sick, in terms of the diagnosis and therefore, has utility as a diagnostic method. A high specificity finding combined with a high NPV means that a ROC test only rarely classifies a person with schizophrenia/psychosis as being free of that diagnosis, and the test therefore has utility as a diagnostic exclusion, screening tool.

### Imputation

In some instances symptomatic participants were unable to complete all the tests in the MIRAT model testing domains. If the data already present in the domain met the cut-off requirements for the cut-off for the composite Nutrition-Biochemistry, Visual or Auditory model respectively, then the value for the domain was imputed and contributed to the score for the 'Combined model with imputed values'. This procedure did not alter the cut-off point from the 'Combined model' with no imputation, and accommodates the real life scenario in which symptomatic patients may be unable to complete all tests due to cognitive or motor deficits. It is possible to apply this same method for imputing the final outcome for the MIRAT model when full data for a patient is not available.

### Clinical Validation

A correlation matrix comprising of spearman correlation coefficients was constructed to identify the relationships between the domains of the MIRAT model and measures of severity (Symptom Intensity Rating (SIR) and Clinical Global Impression (CGI) and disability (GAF) and disability support pension (DSP) , and treatment resistance (number of hospital admissions).

### Example 2 - MIRAT assessment of symptomatic and asymptomatic subjects

Receiver operating characteristic (ROC) analysis and odds ratio analysis was carried out on the variables described above in Example 1 as measured in the 67 selectively medicated symptomatic subjects and 67 asymptomatic subjects described in Example 1.

These analyses identified a number of variables that were capable of differentiating cases from controls by demonstrating an area under the curve and other parameters of sufficient significance to consider them to be biomarkers. These variables were classified into five main categories (domains) and one supplementary category (domain). Summarised values for a five domain model, including the five main domain, and six domain model, including these five main domain and the supplementary domain (middle ear domain), are shown in Table 2. These categories were neurotransmitters, oxidative stress, nutrition, visual processing, auditory processing, and measures of middle ear performance in terms of tympanic reflex and stapes muscle contraction. These biomarker variables were combined to form a combined multi-domain model (called MIRAT Model) of schizophrenia and schizo-affective disorder. Table 3 details the variables selected in the MIRAT model and their statistical parameters.

Odds ratio analysis performed on summated ROC scores of the multiple ROC domains in this model yielded a risk of association with a diagnosis of schizophrenia or schizoaffective disorder measure. Any value greater than 10 for the odds ratio is considered significant. While some variables do not meet this value individually, they nonetheless contribute significantly to the model. It is clear from the odds ratios in Table 2 that combining markers into models gives better statistical outcomes than using single markers.

Individual biomarker variables in the MIRAT model showed either high sensitivity or high specificity for the detection of schizophrenia/psychosis. Noradrenaline, adrenaline, visual span, visual speed of processing variables, competing words, auditory speed of processing variables and low peak middle ear pressure, showed both high sensitivity and high specificity.

Each subcomponent of the 5 and 6 domain MIRAT Model was scored to a one or a zero using its unique cut-off value that was identified through ROC analysis. The subcomponent scores were tallied for the Domain and then the Domain was scored as a one or a zero based on its unique cut-off value that was identified through ROC analysis. Domains with missing subcomponent values but a sufficient subcomponent tally to code the Domain to one or zero were imputed. The scores for each Domain were combined (tallied) to provide a total score for the MIRAT Model. The minimum score is zero and the maximum score is five. The combined MIRAT Model score is used to identify the risk of schizophrenia/psychosis being present and/or developing in the future. Non-parametric and Log-logistic Regression models identify the risk of schizophrenia/psychosis based on a combined MIRAT Model score of 1 through to 5. A combined score of 3 or more abnormal Domains is indicative of a significant risk of diagnosis of schizophrenia/psychosis. The Middle Ear Domain is used in borderline cases to supplement the information provided by the main combined Five Domain Model.

This process of coding and combining individual biomarkers resulting in the combined model with imputation, demonstrated a sensitivity of 73 to 93 per cent and a specificity of 80 to 96 per cent, for identification of the schizophrenia/ psychosis condition, at the 95% level of significance. The model correctly identified 43 out of 50 (86 per cent) of symptomatic participants as schizophrenia/psychosis and 59 out of 65 (91 per cent) of asymptomatic participants as no schizophrenia/psychosis. The additional use of the supplementary Middle Ear Domain enables the identification of 47 out of 49 (96 per cent) of symptomatic participants as schizophrenia/psychosis. Also, there was insignificant difference between imputed and non-imputed form of the model, as shown in Figure 1.

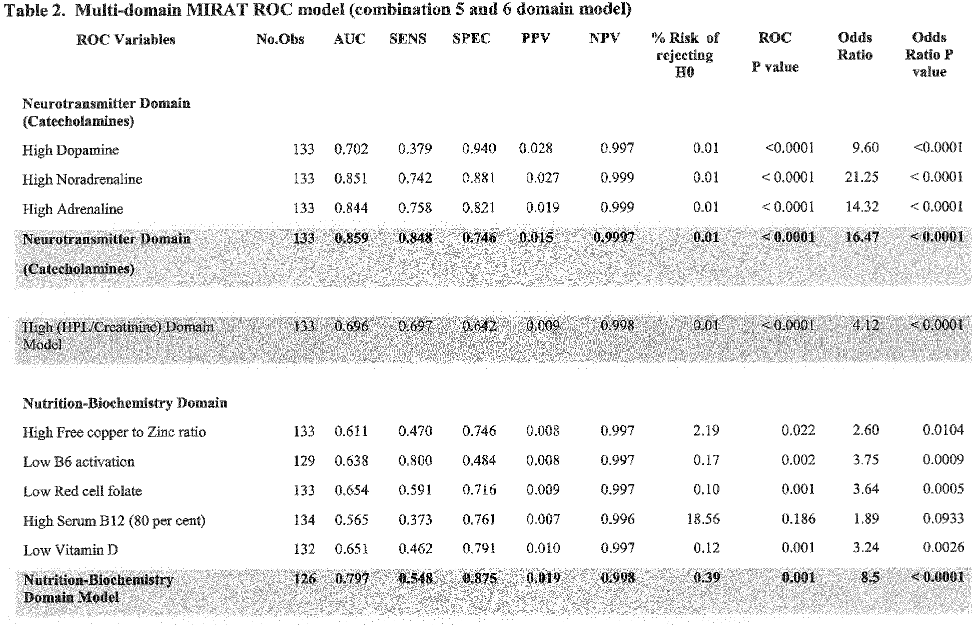

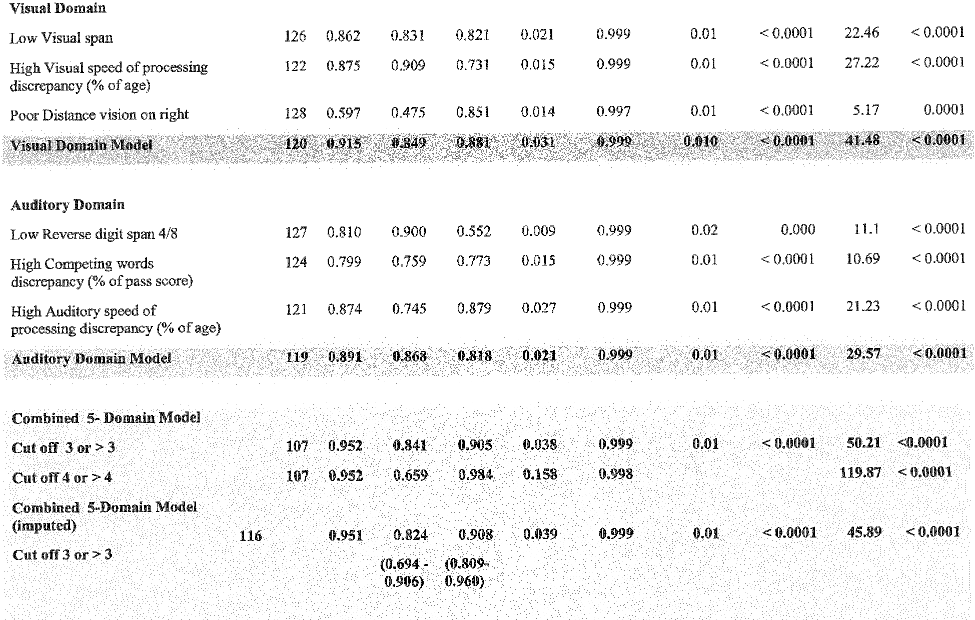

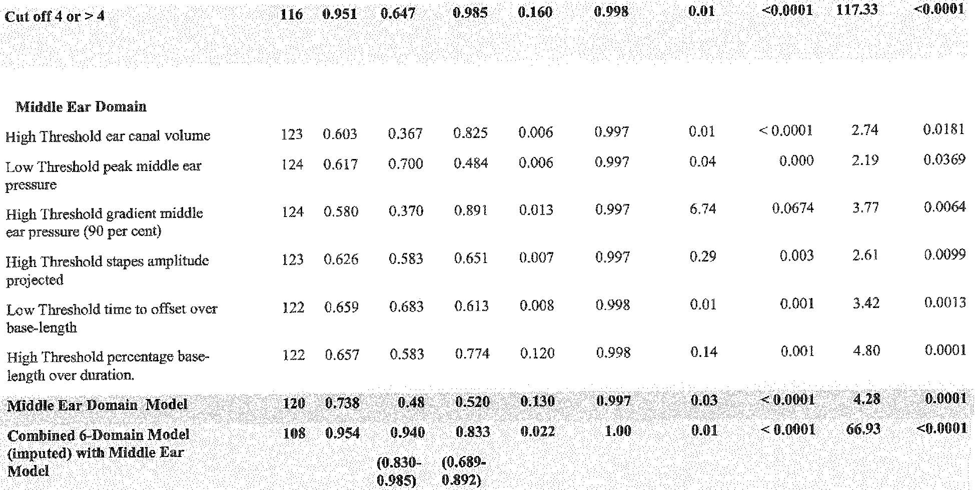

**Table 3. MIRAT multi-domain model data**

| ROC Variables | No Obs | Sensitivity | Lower bound | Upper bound | Specificity | Lower bound | Upper bound | PPV | NPV | LR+ | LR- | TP | FP | FN | Accuracy |
|---|---|---|---|---|---|---|---|---|---|---|---|---|---|---|---|
| **Neurotransmitter Domain** | | | | | | | | | | | | | | | |
| High Dopamine | 133 | 0.3788 | 0.2717 | 0.4997 | 0.9403 | 0.8508 | 0.9802 | 0.0279 | 0.9970 | 6.3447 | 0.6607 | 25 | 4 | 41 | 0.6617 |
| High Noradrenaline | 133 | 0.7424 | 0.6244 | 0.8327 | 0.8806 | 0.7782 | 0.9401 | 0.0273 | 0.9987 | 6.2178 | 0.2925 | 49 | 8 | 17 | 0.8120 |
| High Adrenaline | 133 | 0.7576 | 0.6405 | 0.8452 | 0.8209 | 0.7103 | 0.8954 | 0.0188 | 0.9987 | 4.2298 | 0.2953 | 50 | 12 | 16 | 0.7895 |

| **Neurotransmitter Model** | | | | | | | | | | | | | | | |
|---|---|---|---|---|---|---|---|---|---|---|---|---|---|---|---|
| **Domain** | 133 | 0.8485 | 0.7403 | 0.9169 | 0.7463 | 0.6294 | 0.8354 | 0.0149 | 0.9991 | 3.3440 | 0.2030 | 56 | 17 | 10 | 0.7970 |
| **High (HPL/Creatinine) Model/ Domain** | 133 | 0.6970 | 0.5770 | 0.7945 | 0.6418 | 0.5217 | 0.7459 | 0.0087 | 0.9979 | 1.9457 | 0.4722 | 46 | 24 | 20 | 0.6692 |

| **Nutrition-Biochemistry Domain** | | | | | | | | | | | | | | | |
|---|---|---|---|---|---|---|---|---|---|---|---|---|---|---|---|
| High Free copper to Zinc ratio | 133 | 0.4697 | 0.3545 | 0.5884 | 0.7463 | 0.6294 | 0.8354 | 0.0083 | 0.9968 | 1.8512 | 0.7106 | 31 | 17 | 35 | 0.6090 |
| Low B6 activation | 129 | 0.8000 | 0.6853 | 0.8799 | 0.4844 | 0.3665 | 0.6041 | 0.0070 | 0.9981 | 1.5515 | 0.4129 | 52 | 33 | 13 | 0.6434 |
| Low Red cell folate | 133 | 0.5909 | 0.4703 | 0.7011 | 0.7164 | 0.5981 | 0.8104 | 0.0093 | 0.9974 | 2.0837 | 0.5710 | 39 | 19 | 27 | 0.6541 |
| High Serum B12 (80 per cent) | 134 | 0.3731 | 0.3064 | 0.4541 | 0.7612 | 0.6803 | 0.8126 | 0.0070 | 0.9963 | 1.5625 | 0.8235 | 25 | 16 | 42 | 0.5672 |
| Low Vitamin D | 132 | 0.4615 | 0.3461 | 0.5814 | 0.7910 | 0.6775 | 0.8718 | 0.0099 | 0.9969 | 2.2088 | 0.6807 | 30 | 14 | 35 | 0.6288 |
| **Nutrition-Biochemistry Model/Domain** | 126 | 0.5484 | 0.4253 | 0.6656 | 0.8750 | 0.7688 | 0.9371 | 0.0194 | 0.9977 | 4.3871 | 0.5161 | 34 | 8 | 28 | 0.7143 |

| **Visual Domain** | | | | | | | | | | | | | | | |
|---|---|---|---|---|---|---|---|---|---|---|---|---|---|---|---|
| Low Visual span | 126 | 0.8305 | 0.7126 | 0.9065 | 0.8209 | 0.7103 | 0.8954 | 0.0205 | 0.9991 | 4.6370 | 0.2065 | 49 | 12 | 10 | 0.8254 |
| High Visual speed of processing discrepancy (% of age) | 122 | 0.9091 | 0.8331 | 0.9851 | 0.7313 | 0.6252 | 0.8375 | 0.0151 | 0.9994 | 3.3838 | 0.1243 | 50 | 18 | 5 | 0.8115 |
| Poor Distance vision on right | 128 | 0.4754 | 0.3555 | 0.5983 | 0.8507 | 0.7438 | 0.9182 | 0.0142 | 0.9972 | 3.1852 | 0.6166 | 29 | 10 | 32 | 0.6719 |
| **Visual Model/Domain** | 120 | 0.8491 | 0.7822 | 0.8670 | 0.8806 | 0.8244 | 0.8939 | 0.0311 | 0.9992 | 7.1108 | 0.1714 | 45 | 8 | 8 | 0.8667 |
| **Combined Model Imputed* (cut-off > or = 3)** | 116 | 0.8235 | 0.6943 | 0.9057 | 0.9077 | 0.8085 | 0.9596 | 0.0388 | 0.9991 | 8.9216 | 0.1944 | 42 | 6 | 9 | 0.8707 |
| **Combined Model** | | | | | | | | | | | | | | | 0.8362 |
| **Imputed* (cut-off > or = 4)** | 116 | 0.6471 | 0.5092 | 0.7635 | 0.9846 | 0.9084 | 1.0000 | 0.1597 | 0.9984 | 42.058 8 | 0.3585 | 33 | 1 | 18 | |
| **Combined Model Imputed* (cut-off > or = 5)** | 116 | 0.2745 | 0.1709 | 0.4109 | 1.0000 | 0.9314 | 1.0000 | 1.0000 | 0.9967 | +Inf | 0.7255 | 14 | 0 | 37 | 0.6810 |

| **Supplementary Middle Ear Domain** | | | | | | | | | | | | | | | |
|---|---|---|---|---|---|---|---|---|---|---|---|---|---|---|---|
| High Threshold ear canal volume | 123 | 0.3667 | 7 0.2564 | 0.4936 | 0.8254 | 0.7113 | 0.9007 | 0.0094 | 0.9965 | 2.1000 | 0.7673 | 22 | 11 | 38 | 0.6016 |
| Low Threshold peak middle ear pressure | 124 | 0.7000 | 0.5739 | 0.8011 | 0.4844 | 0.3665 | 0.6041 | 0.0061 | 0.9972 | 1.3576 | 0.6194 | 42 | 33 | 18 | 0.5887 |
| High Threshold gradient middle ear pressure (90 per cent) | 124 | 0.3167 | 0.2316 | 0.4247 | 0.8906 | 0.8001 | 0.9352 | 0.0129 | 0.9965 | 2.8952 | 0.7673 | 19 | 7 | 41 | 0.6129 |
| High Threshold | | | | | | | | | | | | | | | 0.6179 |
| stapes amplitude projected | 123 | 0.5833 | 0.4571 | 0.6991 | 0.6508 | 0.5270 | 0.7566 | 0.0075 | 0.9971 | 1.6705 | 0.6402 | 35 | 22 | 25 | |
| Low Threshold time to offset over base length | 122 | 0.6833 | 0.5568 | 0.7869 | 0.6129 | 0.4882 | 0.7239 | 0.0079 | 0.9977 | 1.7653 | 0.5167 | 41 | 24 | 19 | 0.6475 |
| High Threshold percentage base length over duration | 122 | 0.5833 | 0.4571 | 0.6991 | 0.7742 | 0.6542 | 0.8610 | 0.0115 | 0.9976 | 2.5833 | 0.5382 | 35 | 14 | 25 | 0.6803 |
| **Middle Ear Model/Domain** | 120 | 0.8000 | 0.6800 | 0.8825 | 0.5167 | 0.3932 | 0.6381 | 0.0074 | 0.9983 | 1.6552 | 0.3871 | 48 | 29 | 12 | 0.6583 |

| | | | | | | | | | | | | | | | |
|---|---|---|---|---|---|---|---|---|---|---|---|---|---|---|---|
| All values reported at 95% confidence interval unless otherwise stated. | | | | | | | | | | | | | | | |

When logistic regression analysis was performed on summated ROC scores of the multiple ROC domains in this model, this yielded a predictive risk of schizophrenia or schizo-affective disorder or Mental Illness Risk Assessment Test (MIRAT). In this Mental Illness Risk Prediction Test, percentage risk of schizophrenia diagnosis is presented in relationship to the number of MIRAT domains that were scored as containing ROC biomarker-variables that were significantly abnormal. When Logistic Regression was performed on summated ROC scores of the multiple ROC domains in this model, this yielded the Mental Illness Risk Assessment Test (MIRAT). In this Mental Illness Risk Prediction Test, percentage risk of schizophrenia diagnosis is presented in relationship to the number of MIRAT domains which were scored as containing ROC biomarker-variables that were significantly abnormal. The Nagelkerke R2 for the logistic model of 'Combined model (imputed)' and symptomatic/asymptomatic status was 0.752. A non-parametric regression model was also constructed. The goodness of fit (R2) for the non-parametric regression model was 0.626. Logistic and non-parametric regression models of MIRAT are shown in Table 4.

**Table 4. Predictive relationships using logistic regression and non-parametric regression**

| **Combination 5 domain Model (with imputation).** | | | | | |
|---|---|---|---|---|---|
| Logistic Regression | | | | | Non Parametric (LOWESS) Regression |
| Score | Predicted | Lower bound (%) | | Upper bound (%) | Predicted risk (%) |
| Risk | | | | | |
| | 0 | 0.97 | 0.19 | 4.78 | 0 |
| | 1 | 6.13 | 2.21 | 15.87 | 3.7 |
| | 2 | 30.32 | 18.30 | 45.81 | 38.88 |
| | 3 | 74.37 | 56.99 | 86.40 | 66.22 |
| | 4 | 95.08 | 84.28 | 98.59 | 88.48 |
| | 5 | 99.23 | 95.16 | 99.88 | 100 |

| | | | | | |
|---|---|---|---|---|---|
| *Contains imputed values for Nutrition-Biochemistry, Visual, or Auditory Domains | | | | | |

Incorporating the supplementary Middle Ear domain reduces the number of false negatives from seven to two of the symptomatic participants. It does however increase the number of false positives from six to twelve and so should only be used when schizophrenia/psychosis is suspected but the main MIRAT model returns a negative result for schizophrenia/psychosis.

The odds ratio analysis (Table 5) demonstrates the association of the score for the number of abnormal domains with a diagnosis of schizophrenia or schizo-affective disorder. Odds ratio analysis still demonstrates that abnormality of more than 3 domains of the model is significantly associated with a diagnosis of schizophrenia, schizo-affective disorder or psychosis, as described above. Moreover this model demonstrates that abnormality in 4 domains of the model is associated with a very high risk of having these conditions (as is shown in the previous logistic regression model).

**Table 5. Odds ratio: combined 5 domain model**

| **Number of abnormal domains** | **Odds Ratio TPxTN/(FPxFN)** | **Lower bound 95% confidence interval** | **Upper bound 95% confidence interval** | **Standard error** | **p value** |
|---|---|---|---|---|---|
| 3 | 45.8 | 15.18 | 138.71 | 0.5644 | < 0.0001 |
| 4 | 117.3 | 14.99 | 917.88 | 1.0495 | < 0.0001 |

In order to cross-validate the combined model, a correlation matrix comprising of Spearman correlation coefficients (rho) was constructed. The Combined Model (MIRAT) with imputed values, showed high level correlations with severity and disability, and treatment resistance, at the 95 per cent level of significance (Table 6).

**Table 6 - Spearman rank correlations for functional outcome ratings in relationship to biomarker variables and domain models.**

| Multi-domain model | Case versus Control | SOFAS ROC | GAF ROC | CGI ROC | Treatment resistance | Disability Pension | Symptom intensity Rating (SIR) |
|---|---|---|---|---|---|---|---|
| Combined 5-domain model with imputed values | **0.770** | **0.752** | **0.770** | **0.754** | **0.830** | **0.677** | **0.697** |
| Combined multi-domain model (no imputation) | **0.789** | **0.772** | **0.772** | **0.774** | **0.829** | **0.651** | **0.697** |
| Neurotransmitter domain | **0.598** | **0.591** | **0.562** | **0.591** | **0.583** | **0.460** | **0.467** |
| High HPL/Creatinine | **0.339** | **0.312** | **0.315** | **0.312** | **0.421** | **0.296** | **0.327** |
| Nutrition-Biochemistry domain | **0.458** | **0.415** | **0.415** | **0.415** | **0.403** | **0.309** | **0.404** |
| Visual domain | **0.730** | **0.727** | **0.745** | **0.729** | **0.766** | **0.608** | **0.624** |
| Auditory domain | **0.650** | **0.632** | **0.618** | **0.636** | **0.608** | **0.530** | **0.583** |
| Middle Ear domain | **0.340** | **0.341** | **0.328** | **0.341** | **0.300** | **0.212** | **0.377** |
| Combined (imputed) | **0.775** | **0.758** | **0.742** | **0.761** | **0.748** | **0.609** | **0.636** |
| 6-domain model, (with middle ear domain). | | | | | | | |

| | | | | | | | |
|---|---|---|---|---|---|---|---|
| All correlation coefficient (rho) values are significant at the 95 per cent level of significance. Impact of visual domain biomarkers on adverse functional outcomes is particularly noteworthy. Clinical Global Impression of Severity (CGI), Global Assessment of Function (GAF), Social and Occupational Functioning Assessment Scale (SOFAS). HPL = urinary hydroxyhaemopyrroline-2-one. | | | | | | | |

The MIRAT multi-domain model was also applied to symptoms rated for intensity, from the Brief Psychiatric Rating Scale (BPRS) and the Positive and Negative Symptom Scale (PANSS). Spearman correlation analysis revealed weak, moderate and strong level correlations between the individual ROC components, the ROC domains, the ROC models and the overall combined model (data not shown), forming the basis for a future biological classification system of serious mental illness states, symptoms and behaviours. An exemplary "signature" of the Spearman Rank Correlation Coefficients for the blunted affect schizophrenia symptom is shown in Table 7 (all values significant at p<0.001). Similar signatures can be generated for other symptoms of schizophrenia and schizo-affective disorder including, for example, hallucinations, delusions, suspiciousness, hostility and impulse control.

**Table 7. Example symptom profile: for blunted affect, using Spearman's correlation at 95% significance (alpha 0.05).**

| **Biomarker ROCs** | **Blunted Affect Rho for alpha 0.05** |
|---|---|
| Low Visual Span (n 126) | 0.567 |
| Low Auditory Speed of processing (% of age) (n 121) | 0.548 |
| High Noradrenaline (n 133) | 0.513 |
| Low Competing words score (% of pass score) (n 124) | 0.489 |
| Low Visual speed of processing (% of age) (n 122) | 0.482 |
| High Adrenaline (n 133) | 0.441 |
| Low Reverse Digit Span (n 128) | 0.411 |
| Long Threshold percent base-length/duration (n 122) | 0.294 |
| High Distance vision score (poor vision) on Right (n 128) | 0.283 |
| High HPL/Creatinine (n 133) | 0.273 |
| High Threshold gradient middle ear pressure (n 124) | 0.264 |
| Low Vitamin D (n 132) | 0.233 |
| Low threshold time to offset/base length (n 122) | 0.211 |
| High Threshold Stapes Amplitude Projected (n 123) | 0.205 |
| Low red cell folate (n 133) | 0.193 |
| Low activated vitamin B6 (n 126) | 0.187 |
| High Dopamine (n 133) | 0.182 |

In particular, the inventor was able to find significant correlates for key symptoms and behaviours that have important implications for management in the clinical setting, including insight-and-judgement-impairment, anxiety, auditory hallucinations, depressed mood, motor hyperactivity, hypo-activity, suicidality and aggression.

### Example 3 - Exemplary clinical application of the multi-domain MIRAT model and MIRAT test

The following is provided, by way of example only, as a means of employing a MIRAT test in a clinical setting.

A clinician (such as a general practitioner) registers with a dedicated MIRAT or other named website, submits their credentials, obtains patient consent, and orders patient blood and urine tests. The clinician also completes a symptom check-list and undertakes a number of neuro-sensory and cognitive tests. The results of the blood and urine tests for the patient will be supplied to both the clinician and a central body or authority maintaining and holding MIRAT test information, and will be entered onto the website, together with the results of the cognitive tests.

Two algorithms or calculations are then applied to the test results by the central body or authority holding the MIRAT test information, one algorithm to provide a risk prediction score and diagnostic accuracy for the patient in which scores are obtained over several cumulative domains of patient-functioning on the MIRAT multi-domain model and risk-prediction relies on how many abnormalities exist in each domainat a biomarker threshold level. The other algorithm or calculation is for determining risk prediction and diagnostic accuracy based upon symptom ratings using symptom rating scales comprising the Brief Psychiatric Symptom Rating Scale (BPRS) combined with the Positive and Negative Syndrome Scale (PANSS) symptom ratings. The clinician will then be supplied with an overall outcome table, such as depicted in Tables 4 and 5 (above) and/or a risk prediction index.

In cases which do not reach threshold for psychosis/schizophrenia progression, or to increase the sensitivity of the test, the middle ear domainmay be employed. Alternatively or in addition, evidence or information for an alternative diagnosis such as depression may also be supplied to the clinician.

## Claims

1. A method for diagnosing schizophrenia, schizo-affective disorder and/or psychosis in an individual or predicting risk of the individual for developing schizophrenia, schizo-affective disorder or psychosis, the method comprising:
(i) determining values for one or more markers in each of five domains in one or more biological samples obtained from the individual:
(a) a neurotransmitter domain comprising dopamine, noradrenaline and adrenaline;
(b) an oxidative stress domain comprising urinary hydroxyhemopyrroline-2-one and urinary creatinine, and/or other marker of oxidative stress;
(c) a nutrition-biochemistry domain comprising free copper to zinc ratio, activated vitamin B6, red cell folate, serum vitamin B12, and vitamin D;
(d) a visual processing domain comprising visual span, visual speed of processing discrepancy, visual speed of processing, and distance vision on right; and
(e) an auditory processing domain comprising reverse digit span, competing words discrepancy, auditory speed of processing discrepancy, and auditory speed of processing;
and
(ii) comparing values for said one or more markers in each of said domains to control values of said markers in subjects not suffering from schizophrenia, schizo-affective disorder or psychosis, wherein the values of said markers indicative of schizophrenia or psychosis are, relative to said control values:
- in the neurotransmitter domain, high dopamine, high noradrenaline, and high adrenaline;
- in the oxidative stress domain, high urinary hydroxyhemopyrroline-2-one divided by urinary creatinine;
- in the nutrition-biochemistry domain, high free copper to zinc ratio (or low zinc to free copper ratio), low activated vitamin B6, low red cell folate, high serum vitamin B12, and low vitamin D;
- in the visual processing domain, low visual span, high visual speed of processing discrepancy (percentage of age), low visual speed of processing (percentile), and poor distance vision on right; and
- in the auditory processing domain, low reverse digit span, high competing words discrepancy (percentage of pass score), high auditory speed of processing discrepancy (percentage of age), and low auditory speed of processing (percentile).

2. The method of claim 1, wherein (i) further comprises determining the value of reduced glutathione (GSH); and (ii) further comprises comparing the value for reduced glutathione (GSH) to a control value of reduced glutathione (GSH) in subjects not suffering from schizophrenia, schizo-affective disorder or psychosis, wherein the value of reduced glutathione (GSH) indicative of schizophrenia or psychosis is low relative to said control value.

3. The method of claim 1 or claim 2, wherein (i) further comprises determining the value of oxidised glutathione (GSSG); and (ii) further comprises comparing the value for oxidised glutathione (GSSG) to a control value of oxidised glutathione (GSSG) in subjects not suffering from schizophrenia, schizo-affective disorder or psychosis, wherein the value of oxidised glutathione (GSSG) indicative of schizophrenia or psychosis is high relative to said control value.

4. The method of claim 2 or claim 3, wherein low reduced glutathione and high oxidised glutathione in the one or more biological samples obtained from the individual, compared to levels in subjects not suffering from schizophrenia, schizo-affective disorder or psychosis, are suggestive of schizophrenia, schizo-affective disorder or psychosis in the individual.

5. The method of any one of claims 1 to 4, comprising determining values for each of said markers in each of said domains.

6. The method of any one of claims 1 to 5, further comprising determining values for one or more markers in a middle ear domain comprising threshold ear canal volume, threshold peak middle ear pressure, threshold gradient middle ear pressure, threshold stapes amplitude projected, threshold time to offset divided by baselength and threshold percentage baselength divided by duration, and comparing values for said one or more markers to control values of said markers in subjects not suffering from schizophrenia or psychosis, wherein the values of said markers indicative of schizophrenia or psychosis are, relative to said control values, high threshold ear canal volume, low threshold peak middle ear pressure, high threshold gradient middle ear pressure, high threshold stapes amplitude projected, low threshold time to offset divided by baselength and high threshold percentage baselength divided by duration.

7. The method of any one of claims 1 to 6, wherein said method comprises conducting statistical analysis of determined values of said markers in combination and diagnosing schizophrenia or psychosis in said individual on the basis of combined analysis.

8. The method of any one of claims 1 to 7, wherein said statistical analysis comprises receiver operating characteristic (ROC) analysis and/or odds ratio analysis.

9. The method of claim 8, wherein said ROC analysis comprises ascertaining ROC ranges for individual ROC variables and combined or summated sets of ROC variables, using an appropriate means to determine standard deviation adjusted for the position of ROC-variable-cut-off values in the distribution of their variable ranges.

10. The method of claim 8 or 9, wherein summated ROC scores of multiple ROC domains are subjected to odds-ratio analysis or logistic regression, for the purpose of determining diagnosis accuracy or risk prediction.

## Patentansprüche

1. Verfahren zur Diagnose von Schizophrenie, schizoaffektiver Störung und/oder Psychose bei einem Individuum oder zur Vorhersage des Risikos eines Individuums, Schizophrenie, schizoaffektive Störung oder Psychose zu entwickeln, wobei das Verfahren Folgendes umfasst:
(i) Bestimmung von Werten für einen oder mehrere Marker in jeder von fünf Domänen in einer oder mehreren von dem Individuum gewonnenen biologischen Proben:
(a) eine Neurotransmitter-Domäne umfassend Dopamin, Noradrenalin und Adrenalin;
(b) eine oxidative Stress-Domäne umfassend Urin-Hydroxyhämopyrrolin-2-on und Urin-Kreatinin und/oder andere Marker für oxidativen Stress;
(c) eine Ernährungs-Biochemie-Domäne umfassend das Verhältnis von freiem Kupfer zu Zink, aktiviertes Vitamin B6, Erythrozytenfolat, Serumvitamin B12 und Vitamin D;
(d) eine visuelle Verarbeitungsdomäne umfassend visuelle Spanne, Diskrepanz der visuellen Verarbeitungsgeschwindigkeit, visuelle Verarbeitungsgeschwindigkeit und Fernsicht rechts; und
(e) eine auditive Verarbeitungsdomäne umfassend Reverse Digit Span, Competing Words-Diskrepanz, Diskrepanz der auditiven Verarbeitungsgeschwindigkeit und auditive Verarbeitungsgeschwindigkeit;
und
(ii) Vergleich von Werten dieses einen oder dieser mehreren Marker in jeder dieser Domänen mit Kontrollwerten dieser Marker bei Subjekten, die nicht an Schizophrenie, schizoaffektiver Störung oder Psychose leiden, wobei sich die Werte dieser Marker, die auf Schizophrenie oder Psychose schließen lassen, im Verhältnis zu den Kontrollwerten folgendermaßen verhalten:
- in der Neurotransmitter-Domäne hohes Dopamin, hohes Noradrenalin und hohes Adrenalin;
- in der oxidativen Stress-Domäne hohes Urin-Hydroxyhämopyrrolin-2-on geteilt durch Urin-Kreatinin;
- in der Ernährungs-Biochemie-Domäne hohes Verhältnis von freiem Kupfer zu Zink (oder niedriges Verhältnis von Zink zu freiem Kupfer), niedriges aktiviertes Vitamin B6, niedriges Erythrozytenfolat, hohes Serumvitamin B12 und niedriges Vitamin D;
- in der visuellen Verarbeitungsdomäne geringe visuelle Spanne, hohe Diskrepanz der visuellen Verarbeitungsgeschwindigkeit (Altersanteil), geringe visuelle Verarbeitungsgeschwindigkeit (Perzentil) und schlechte Fernsicht rechts; und
- in der auditiven Verarbeitungsdomäne geringe Reverse Digit Span, hohe Competing Words-Diskrepanz (Anteil der Mindestpunktzahl zum Bestehen), hohe Diskrepanz der auditiven Verarbeitungsgeschwindigkeit (Altersanteil) und geringe auditive Verarbeitungsgeschwindigkeit (Perzentil).

2. Verfahren nach Anspruch 1, wobei (i) des Weiteren die Bestimmung des Wertes von reduziertem Glutathion (GSH) umfasst; und (ii) des Weiteren den Vergleich des Werts von reduziertem Glutathion (GSH) mit einem Kontrollwert von reduziertem Glutathion (GSH) bei Subjekten, die nicht an Schizophrenie, schizoaffektiver Störung oder Psychose leiden, umfasst, wobei der Wert von reduziertem Glutathion (GSH), der auf Schizophrenie oder Psychose schließen lässt, im Vergleich zu dem Kontrollwert gering ist.

3. Verfahren nach Anspruch 1 oder Anspruch 2, wobei (i) des Weiteren die Bestimmung des Wertes von oxidiertem Glutathion (GSSG) umfasst; und (ii) des Weiteren den Vergleich des Werts von oxidiertem Glutathion (GSSG) mit einem Kontrollwert von oxidiertem Glutathion (GSSG) bei Subjekten, die nicht an Schizophrenie, schizoaffektiver Störung oder Psychose leiden, umfasst, wobei der Wert von oxidiertem Glutathion (GSSG), der auf Schizophrenie oder Psychose schließen lässt, im Vergleich zu dem Kontrollwert hoch ist.

4. Verfahren nach Anspruch 2 oder Anspruch 3, wobei geringes reduziertes Glutathion und hohes oxidiertes Glutathion in einer oder mehreren von dem Individuum entnommenen biologischen Proben im Vergleich mit Werten von Subjekten, die nicht an Schizophrenie, schizoaffektiver Störung oder Psychose leiden, auf Schizophrenie, schizoaffektive Störung oder Psychose bei dem Individuum schließen lassen.

5. Verfahren nach einem der Ansprüche 1 bis 4, umfassend die Bestimmung von Werten für jeden der besagten Marker in jeder der besagten Domänen.

6. Verfahren nach einem der Ansprüche 1 bis 5, weiter umfassend die Bestimmung von Werten für einen oder mehrere Marker in einer Mittelohrdomäne, umfassend Schwellenwert Gehörgangsvolumen, Schwellenwert Spitzendruck Mittelohr, Schwellenwert Gradient Mittelohrdruck, Schwellenwert projizierte Steigbügelamplitude, Schwellenwert Zeit zum Ausgleich geteilt durch die Basislänge und Schwellenwert Prozentsatz der Basislänge geteilt durch die Dauer, sowie den Vergleich von Werten für diesen einen oder diese mehreren Marker mit Kontrollwerten dieser Marker bei Subjekten, die nicht an Schizophrenie oder Psychose leiden, wobei die Werte dieser Marker, die auf Schizophrenie oder Psychose schließen lassen, im Vergleich zu diesen Kontrollwerten hoher Schwellenwert Gehörgangsvolumen, geringer Schwellenwert Spitzendruck Mittelohr, hoher Schwellenwert Gradient Mittelohrdruck, hoher Schwellenwert projizierte Steigbügelamplitude, geringer Schwellenwert Zeit zum Ausgleich geteilt durch die Basislänge und hoher Schwellenwert Prozentsatz der Basislänge geteilt durch die Dauer sind.

7. Verfahren nach einem der Ansprüche 1 bis 6, wobei dieses Verfahren die Durchführung einer statistischen Analyse von ermittelten Werten dieser Marker in Kombination und die Diagnose der Schizophrenie oder Psychose bei dem Individuum auf Basis einer kombinierten Analyse umfasst.

8. Verfahren nach einem der Ansprüche 1 bis 7, wobei diese statistische Analyse die Receiver-Operating-Characteristic (ROC)-Analyse und/oder die Odds-Ratio-Analyse umfasst.

9. Verfahren nach Anspruch 8, wobei diese ROC-Analyse die Ermittlung von ROC-Wertebereichen für einzelne ROC-Variablen und kombinierte oder summierte ROC-Variablensets umfasst, wobei ein angemessenes Mittel zur Bestimmung der Standardabweichung, um die Position der ROC-Variablen-Grenzwerte bereinigt, bei der Verteilung ihrer Variablenbereiche eingesetzt wird.

10. Verfahren nach Anspruch 8 oder 9, wobei summierte ROC-Ergebnisse mehrerer ROC-Domänen einer Odds-Ratio-Analyse oder logistischen Regression unterzogen werden, um die Genauigkeit der Diagnose oder die Risikovorhersage zu bestimmen.

## Revendications

1. Méthode pour diagnostiquer la schizophrénie, le trouble schizo-affectif et/ou la psychose chez un individu ou prédire le risque pour l'individu de développer une schizophrénie, un trouble schizo-affectif ou une psychose, la méthode comprenant:
(i) la détermination des valeurs pour un ou plusieurs marqueurs dans chacun des cinq domaines dans un ou plusieurs échantillons biologiques prélevés sur l'individu :
(a) un domaine de neurotransmetteur comprenant la dopamine, la noradrénaline et l'adrénaline;
(b) un domaine de stress oxydatif comprenant de l'hydroxyhémopyrroline-2-one urinaire et de la créatinine urinaire, et/ou autres marqueurs du stress oxydatif;
(c) un domaine nutritionnel-biochimique comprenant un rapport cuivre/zinc libre, de la vitamine B6 activée, du folate de globules rouges, de la vitamine B12 sérique et de la vitamine D;
(d) un domaine de traitement visuel comprenant le champ visuel, la divergence de la vitesse de traitement visuelle, la vitesse de traitement visuelle et la vision à distance à droite; et
(e) un domaine de traitement auditif comprenant l'empan de chiffres inversé, la divergence de mots concurrents, la divergence de traitement de la vitesse auditive et la vitesse de traitement auditive;
et
(ii) la comparaison des valeurs pour ledit ou lesdits marqueurs dans chacun desdits domaines aux valeurs de contrôle desdits marqueurs chez des sujets ne souffrant pas de schizophrénie, de trouble schizo-affectif ou de psychose, les valeurs desdits marqueurs indiquant la schizophrénie ou la psychose étant, relatives auxdites valeurs de contrôle :
- dans le domaine des neurotransmetteurs, de la dopamine élevée, de la noradrénaline élevée et de l'adrénaline élevée;
- dans le domaine du stress oxydant, de l'hydroxyhémopyrroline-2-one urinaire élevée divisée par la créatinine urinaire;
- dans le domaine nutritionnel-biochimique, un rapport cuivre/zinc libre élevé (ou un rapport zinc/cuivre libre faible), une faible teneur en vitamine B6 activée, un faible taux de folate de globules rouges, une teneur élevée en vitamine B12 sérique et une faible teneur en vitamine D;
- dans le domaine du traitement visuel, un faible champ visuel, une divergence élevée de la vitesse de traitement visuelle (pourcentage de l'âge), une faible vitesse de traitement visuelle (percentile) et une mauvaise vision à distance à droite; et
- dans le domaine du traitement auditif, un faible empan de chiffres inversé, une divergence élevée de mots concurrents (pourcentage de réussite), une divergence élevée du traitement de la vitesse auditive (pourcentage d'âge) et une faible vitesse auditive de traitement (percentile).

2. Méthode selon la revendication 1, (i) comprenant en outre la détermination de la valeur du glutathion réduit (GSH); et (ii) comprenant en outre la comparaison de la valeur pour le glutathion réduit (GSH) à une valeur de contrôle du glutathion réduit (GSH) chez des sujets ne souffrant pas de schizophrénie, de trouble schizo-affectif ou de psychose, la valeur du glutathion réduit (GSH) indiquant la schizophrénie ou la psychose étant faible par rapport à ladite valeur de contrôle.

3. Méthode selon la revendication 1 ou 2, (i) comprenant en outre la détermination de la valeur du glutathion oxydé (GSSG); et (ii) comprenant en outre la comparaison de la valeur pour le glutathion oxydé (GSSG) à une valeur de contrôle du glutathion oxydé (GSSG) chez des sujets ne souffrant pas de schizophrénie, de trouble schizo-affectif ou de psychose, la valeur du glutathion oxydé (GSSG) indiquant la schizophrénie ou la psychose étant élevée par rapport à ladite valeur de contrôle.

4. Méthode selon la revendication 2 ou 3, le glutathion réduit faible et le glutathion oxydé élevé dans le ou les échantillons biologiques prélevés sur l'individu, comparés aux taux des sujets ne souffrant pas de schizophrénie, de trouble schizo-affectif ou de psychose, suggérant une schizophrénie, un trouble schizo-affectif ou une psychose chez l'individu.

5. Méthode selon l'une quelconque des revendications 1 à 4, comprenant la détermination des valeurs pour chacun desdits marqueurs dans chacun desdits domaines.

6. Méthode selon l'une quelconque des revendications 1 à 5, comprenant en outre la détermination des valeurs pour un ou plusieurs marqueurs dans un domaine de l'oreille moyenne, comprenant le volume seuil du conduit auditif, la pression seuil crête de l'oreille moyenne, la pression seuil gradient de l'oreille moyenne, l'amplitude seuil d'étrier projetée, le temps seuil avant compensation divisé par la longueur de base et la longueur de base de pourcentage seuil divisée par la durée, et à comparer les valeurs pour ledit ou lesdits marqueurs aux valeurs de contrôle desdits marqueurs chez des sujets ne souffrant pas de schizophrénie ou de psychose, les valeurs desdits marqueurs indiquant la schizophrénie ou la psychose étant, par rapport auxdites valeurs de contrôle, un volume seuil élevé du conduit auditif, une pression seuil crête basse de l'oreille moyenne, une pression seuil de gradient élevée de l'oreille moyenne, une amplitude seuil d'étrier projetée élevée, un temps seuil avant compensation faible divisé par la longueur de base et la longueur de base élevée de pourcentage seuil divisée par la durée.

7. Méthode de l'une quelconque des revendications 1 à 6, ladite méthode comprenant une analyse statistique des valeurs déterminées desdits marqueurs en combinaison et à diagnostiquer la schizophrénie ou la psychose chez ledit individu sur la base de l'analyse combinée.

8. Méthode selon l'une quelconque des revendications 1 à 7, ladite analyse statistique comprenant l'analyse de caractéristique de fonctionnement du récepteur (ROC) et/ou l'analyse de rapport des cotes.

9. Méthode selon la revendication 8, ladite analyse ROC comprenant la vérification des plages ROC des variables ROC individuelles et les ensembles combinés ou totalisés des variables ROC, à l'aide d'un moyen approprié pour déterminer l'écart-type ajusté pour la position des valeurs seuils variables ROC dans la distribution de leurs plages variables.

10. Méthode selon la revendication 8 ou 9, les résultats ROC totalisés des multiples domaines ROC étant soumis à une analyse de rapport de cotes ou à une régression logistique pour déterminer la précision du diagnostic ou la prédiction de risques.
